# EUROPEAN PATENT SPECIFICATION

(11) **EP 1 465 996 B1**
(45) Date of publication and mention of the grant of the patent: **05.11.2008**
(21) Application number: 02793099.9
(22) Date of filing: 20.12.2002
(51) Int. Cl.: C12N 15/12, C07K 14/47, A61K 38/17, C12N 15/62

(54) **RAF/RAS BINDING COMPOUNDS**
RAF/RAS-BINDENDE VERBINDUNGEN
COMPOSES DE LIAISON RAF/RAS

(30) Priority: 21.12.2001 EP 01000788
(43) Date of publication of application: 13.10.2004
(73) Proprietor: Laboratoires Serono SA, 1267 Coinsins, Vaud (CH)
(72) Inventor: RICCARDI, Carlo, I-06100 Perugia (IT)
(74) Representative: Tagliafico, Giulia
(86) International application number: PCT/EP2002/014663
(87) International publication number: WO 2003/054193

(56) References cited:
- EP-A- 0 884 385
- AYROLDI EMIRA ET AL: "Glucocorticoid-induced leucine zipper inhibits the Raf-extracellular signal-regulated kinase pathway by binding to Raf-1." MOLECULAR AND CELLULAR BIOLOGY, vol. 22, no. 22, November 2002 (2002-11), pages 7929-7941, XP002243415 November, 2002 ISSN: 0270-7306
- MITTELSTADT P R ET AL: "Inhibition of AP-1 by the glucocorticoid-inducible protein GILZ." THE JOURNAL OF BIOLOGICAL CHEMISTRY. UNITED STATES 3 AUG 2001, vol. 276, no. 31, 3 August 2001 (2001-08-03), pages 29603-29610, XP002201825 ISSN: 0021-9258 cited in the application
- KOLCH W: "Meaningful relationships: the regulation of the Ras/Raf/MEK/ERK pathway by protein interactions." THE BIOCHEMICAL JOURNAL. ENGLAND 15 OCT 2000, vol. 351 Pt 2, 15 October 2000 (2000-10-15), pages 289-305, XP002201826 ISSN: 0264-6021 cited in the application
- DADAMIO F ET AL: "A new dexamethasone-induced gene of the leucine zipper family protects T lymphocytes from TCR/CD3-activated cell death" IMMUNITY, CELL PRESS, US, XP002078136 ISSN: 1074-7613

### SEQUENCE LISTING

<110> Applied Research Systems ARS Holding N.V.
<120> Novel Raf /Ras binding compounds
<130> WO529
<160> 3
<170> PatentIn version 3.0
<210> 1
   <211> 97
   <212> PRT
   <213> Mus musculus
<400> 1
   Met Asn Thr Glu Met Tyr Gln Thr Pro Met Glu Val Ala Val Tyr Gln
   1               5                   10                  15
   Leu His Asn Phe Ser Thr Ser Phe Phe Ser Ser Leu Leu Gly Gly Asp
               20                  25                  30
   Val Val Ser Val Lys Leu Asp Asn Ser Ala Ser Gly Ala Ser Val Val
           35                  40                  45
   Ala Leu Asp Asn Lys Ile Glu Gln Ala Met Asp Leu Val Lys Asn His
       50                  55                  60
   Leu Met Tyr Ala Val Arg Glu Glu Val Glu Val Leu Lys Glu Gln Ile
   65                  70                  75                  80
   Arg Glu Leu Leu Glu Lys Asn Ser Gln Leu Glu Arg Glu Asn Thr Leu
                   85                  90                  95
   Leu
<210> 2
   <211> 97
   <212> PRT
   <213> Homo sapiens
<400> 2
   Met Asn Thr Glu Met Tyr Gln Thr Pro Met Glu Val Ala Val Tyr Gln
   1 5 10 15
   Leu His Asn Phe Ser Ile Ser Phe Phe Ser Ser Leu Leu Gly Gly Asp
   20 25 30
   Val Val Ser Val Lys Leu Asp Asn Ser Ala Ser Gly Ala Ser Val Val
   35 40 45
   Ala Ile Asp Asn Lys Ile Glu Gln Ala Met Asp Leu Val Lys Asn His
   50 55 60
   Leu Met Tyr Ala Val Arg Glu Glu Val Glu Ile Leu Lys Glu Gln Ile
   65 70 75 80
   Arg Glu Leu Val Glu Lys Asn Ser Gln Leu Glu Arg Glu Asn Thr Leu
   85 90 95
   Leu
<210> 3
   <211> 21
   <212> PRT
   <213> Mus musculus
<400> 3
   Gln Leu His Asn Phe Ser Thr Ser Phe Phe Ser Ser Leu Leu Gly Gly
   1 5 10 15
   Asp Val Val Ser Val
   20

## Description

### FIELD OF THE INVENTION

The present invention concerns the activities of GILZ protein and of GILZ protein-derived compounds in the field of signal transduction.

### BACKGROUND OF THE INVENTION

The efficacy of Glucocorticoid Hormones (GCHs) as therapeutic agents for many acute/chronic inflammatory and autoimmune diseases is, at least partly, due to the effect of GCHs on T cell development and function. The properties of these cells are regulated by a number of stimuli having direct or indirect consequences on the coordinated expression of a number of genes involved in activation and clonal expansion, such as interleukin-2/interleukin-2 receptor. Thymic epithelial cells produce GCHs and it has been proposed that these locally produced glucocorticoids participate in antigen-specific thymocyte development by inhibiting activation-induced gene transcription (Ashwell JD et al., 2000).

GCHs induce apoptosis in thymocytes and activated mature T cells, possibly helping the elimination of developing lymphocytes that are differentiating improperly including neoplastic lymphocytes (Ramdas J and Harmon JM, 1998). Paradoxically, GCHs may also promote survival of thymocytes (Vacchio MS et al., 1994) and, in the periphery, they inhibits the apoptosis induced by continuously antigen-stimulated T lymphocytes, (Activation Induced Cell Death, AICD). This dual effect, induction or inhibition of apoptosis, implies a functional cross talk between two distinct signalling systems, and suggests that the integration of multiple signals, not only of particularly importance for Glucocorticoid Receptor (GR) signal transduction, but also for other biological effects (Jamieson C and Yamamoto KR, 2000). Transcription factors play a fundamental role in this complex regulatory mechanism since the immunosuppressive effects of GCHs arise largely by inhibition of cytokine gene expression, possibly interfering between the GR and transcription factors, as shown for nuclear factor kB (NF-kB) or AP-1 (Jehn BM and Osborne BA, 1997).

Being GCHs such important regulators of T-cell development, many investigators are trying to identify genes whose expression is strictly regulated by GCHs. These researches should provide alternative means to regulate the molecular mechanisms of T cells, overcoming at same time the limitations and the unwanted effects of GCHs, studying for example the effects on transcriptional activity in cells treated with dexamethasone (DEX) a very stable and potent GCHs analogue commonly used in experimental models (Feng A et al., 1995).

Amongst those DEX-induced genes, the Glucocorticoid-Induced Leucine Zipper (GILZ) has been identified as a novel member of the Leucine zipper family whose expression is up-regulated by DEX and down-regulated by T cell receptor (TCR) triggering (WO 98/49291; EP 884385 A1; D'Adamio F et al., 1997; Cannarile L et al., 2001).

Mouse GILZ (mGILZ, SWISSPROT Acc. No. Q9Z2S7), which was initially cloned by comparing mRNA species expressed in DEX-treated and untreated murine thymocytes, is encoded by an mRNA of 1972 nucleotides, with the open reading frame starting at position 206, and contains 137 amino acids. Human GILZ (hGILZ, SWISSPROT Acc. No. Q99576), which has been cloned by homology with mGILZ, is encoded by an mRNA of 1946 nucleotides, with the coding sequence starting at position 241, and contains 134 amino acids.

GILZ protein sequence is homologous with other members of the Leucine zipper family, in particular DIP (Sillard R et al., 1993; Vogel P et al., 1996), TSC-22 (Jay P et al., 1996; Shibanuma M et al., 1992) and thg-1 (Fiorenza MT et al., 2001). These proteins constitute a specific group of Leucine zipper proteins (sometimes called TSC-22/DIP family) whose function has not been clearly defined (Kester HA et al., 1999). GILZ and GILZ-like proteins have been disclosed elsewhere, and described under other names, for example HT22L proteins (WO 98/50425) or SEQ ID NO: 35 (WO 00/77255), and present a central domain containing the Leucine zipper allowing the dimerization, which divides the N-terminal protein domain from the proline-rich C-terminal domain.

GILZ has been found expressed in normal T lymphocytes in the thymus, spleen, and lymph nodes, splenic B cells, and peritoneal macrophages. GILZ gene expression is strongly upregulated by DEX treatment in all those cells. In particular, GILZ is up-regulated by DEX treatment in mouse and human T lymphocytes and is down-regulated by treatment with α-CD3 antibody (in mouse cells) or phytohemagglutinin (in human cells). These results indicate that T-cell activation decreases GILZ expression and suggest that the two events (GILZ expression and T cell activation) might be mutually exclusive (Riccardi C et al., 2000).

As evaluated by Northern blot analysis, there are also non-lymphoid tissues poorly expressing GILZ are brain, kidney and liver. Recently, various articles described the altered expression of GILZ in different cells and tissues, such as primary osteosarcoma cells (Khanna C et al., 2001) or shear stressed human umbilical vein endothelial cells (McCormick SM et al., 2001).

GILZ over-expressing cells show that this protein is able to move into the nucleus and to protect T cells from TCR-activated apoptosis, but not from other apoptotic stimuli, mimicking the functional unresponsiveness and other effects of GCHs that have been described involved in the GCH-mediated immunosuppressive and antiinflammatory activity. This anti-apoptotic effect correlates with the inhibition of activation-induced Fas/FasL and interleukin-2/interleukin-2 receptor up-regulation, has been associated to the GILZ property of binding NF-kB, blocking consequently the nuclear translocation and DNA binding of the NF-kB subunits, without affecting I-kB phosphorylation and degradation or I-kB/NF-kB binding (Ayroldi E et al., 2001).

It has also been reported that GILZ expression inhibits the induction of reporter constructs driven by the FasL, AP-1, NF-AT, or IL-2 promoters (Mittelstadt PR and Ashwell JD, 2001). GILZ was shown capable to interact with c-Fos and c-Jun, inhibiting the NFAT/AP-1- driven transcription. Both c-Fos and c-Jun were efficiently retained by the N-terminal portion of GILZ (residues 1-60), which lacks the leucine zipper, while the C-terminal portion of GILZ (residues 61-137) retained the ability to homodimerize, even though it is not excluded the possibility of heterodimerazation with other Leucine zipper proteins. Alternatively, some studies have been peformed on the homologous protein TSC-22 making use of various deletion mutants to characterize the cellular localization and the effect on its functions (Hino S et al., 2000; Hino S et al., 2002). However, prior art, including the patetn applications originally disclosing human and murine GILZ sequences (WO 98/49291; EP 884385 A1), does not provide any structure-function relationship for GILZ interactions in the field of signal tranduction, and/or for specific GILZ subsequence smaller than major protein domains (N-terminal domain, central leucine zipper, proline-rich C-terminal domain).

AP-1 is regulated, at the level of Jun and Fos transcription and at level of post-translation modification. Both phenomena, as many others, are under control of Raf signal transduction cascade pathway (Weinstein-Oppenheimer CR et al., 2000). Raf-1 (commonly cited simply as "Raf") is a proto-oncogene belonging to a family of Serine/Threonine kinases, to which A-Raf and B-Raf also belong, having distinct tissue distribution and regulation. Raf kinases can phosphorilate and activate, with different efficacy, the Mitogen activated/Extracellular regulated kinases 1 and 2 (MEK-1/-2), which in turn activate mitogen-activated protein kinase (MAPKs) and extracellular signal-regulated kinases (ERKs), leading to the propagation of the signal. Depending on specific stimuli and cellular environment, the Raf-MEK-ERK cascade regulates diverse cellular processes such as proliferation, differentiation, and apoptosis.

Raf was initially identified as a protein reversibly interacting with Ras, a small GTP-binding protein and well studied proto-oncogene. The activation of Ras initiates a complex array of signal transduction events, typical of higher eukaryotes and initiated by receptor and non-receptor tyrosine kinases requiring Raf in order to transduce growth and differentiation signals.

Raf is the Ras substrate and effector best characterized so far, and this kinase is considered as a central component in the signaling pathways involved in normal cell growth and differentiation. Active Ras stimulates, through MAPKs pathway, the phosphorylation and activation of ELK-1 that, in turn, induces transcription of c-Fos and JunB genes. The regulation of Raf activity appears very complex due to the high number of Raf interactions identified so far. A survey of the literature on Raf-associated and/or Raf-affecting molecules reveals several categories of molecules possibly interacting with Raf, including G-proteins, adaptors, chaperons, phosphatases, receptors, kinases, phospholipids (Kolch W, 2000).

Raf and the MAPKs pathway play a fundamental role in the T cell growth and differentiation (Rincon M, 2001), but many evidences suggest that their action is coordinated with the action of GCHs. Glucocorticoid Receptor can repress transactivation to AP-1 and NF-kB without the binding of GR to DNA (De Bosscher K et al., 1997), but GR and Raf can be found as well within the same protein complex (Widen C et al., 2000). Therefore, GR/Raf interaction may be responsible of inhibition of MAPK pathway, which can be achieved also with low concentrations of DEX (Rider LG et al., 1996).

In a large number of human cancers, Ras is locked in its GTP-bound form as a consequence of mutations, leading to constitutive signaling. Thus, the Ras pathway no longer requires an upstream growth signal, and Ras downstream components, such as Raf and ERK-1/-2, are constitutively activated. This process causes cell transforming phenotypes, such as lost of contact growth inhibition, growing in semi-solid medium, and increased proliferation rate. Abnormal expression and/or mutations of Ras and Raf have been shown to trigger these transformed phenotypes caused by the interaction of Ras with Raf. Means to modulate Raf activity and interactions may allow a control on the downstream signal transduction pathway that induces proliferation or differentiation to treat cancers or inhibit metastasis (Kloog Y and Cox AD, 2000).

Thus, Raf interactions, in particular with Ras, have been intensively studied to elucidate the binding determinants and the functional consequences. The aim of these researches is to help the development of molecules, directed either to Ras or to Raf, potentially useful in cancer therapeutics. For example, various mutants and peptides, derived from Ras or Raf, or obtained from computational and structural methods, have been disclosed in the literature as being inhibitor of the Ras/Raf interactions and / or signaling activities (WO 97/34146; Barnard D et al., 1998; Zeng J et al., 2001; Williams JG et al., 2000; Maruta H et al., 2002; Ohnishi M et al., 1998; Winkler DG et al., 1998; Radziwill G et al., 1996; Block C et al., 1996).

Since the Raf/Ras activated signaling pathway is deeply involved in control of cell proliferation and oncogenic transformation, it would be desirable to identify physiologically active molecules binding Raf/Ras and inhibiting this activation. Such Raf/Ras interacting agents could be administered to a human or veterinary patient in a pharmaceutically acceptable form and in a therapeutically effective dosage for prophylaxis and therapy of pathological conditions related to elevated or prolonged Raf/Ras mediated signaling activity.

### SUMMARY OF THE INVENTION

It has now been discovered that the GILZ protein interacts directly with Raf/Ras complex, inhibiting the activation of the signaling pathway controlled by such complex. More specifically, it has now been found that a specific segment in the N-terminal region of GILZ interacts with Raf. These evidences can be exploited to use GILZ, and more particularly N-terminal segments of GILZ, as well as peptides and other molecules designed on the sequence and the structure of the N-terminal domain of GILZ protein.

Compounds prepared in accordance with the present invention can be used to inhibit the intracellular activation of Ras/Raf in cells expressing this protein, thereby providing useful therapeutic compositions for use in the treatment of diseases related to excessive or constitutive activation of Raf/Ras-related signal transduction, as cancers. Other features and advantages of the invention will be apparent from the following detailed description.

### DESCRIPTION OF THE FIGURES

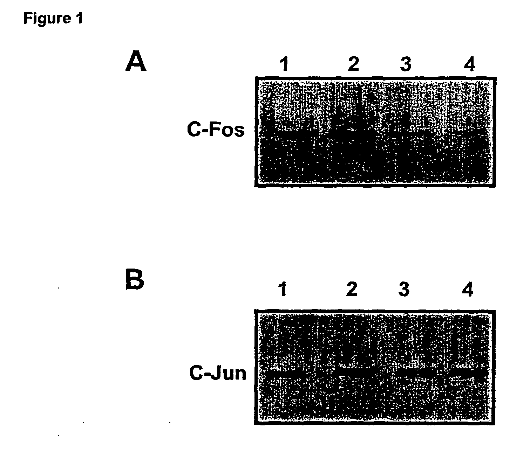

- Figure 1:: Western blot analysis showing the effect of GILZ over-expression on C-Fos (A) and c-Jun (B) expression. Nuclear cell lysates were prepared from the pcDNA3-3DO cells (empty vector transfected clone PV6, lanes 1-2) and GILZ-pcDNA3-3DO cells (clone GIRL-19, lanes 3-4) after being unstimulated (lanes 1 and 3) or stimulated for two hours with immobilised α-CD3 antibodies (lanes 2 and 4).

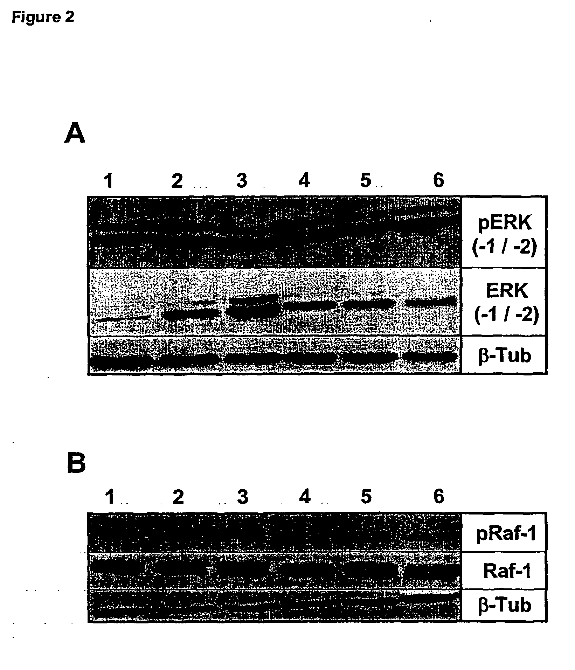

- Figure 2:: Western blot analysis showing the effect of GILZ over-expression on Raf and ERK-1/-2 phosphorylation. Whole cell lysates were prepared from pcDNA3-3DO cells (empty vector transfected clone PV6, lanes 1-3) or GILZ-pcDNA3 3DO cells (clone GIRL-19, lanes 4-6) after being unstimulated (lanes 1 and 4), or stimulated for 20 minutes (lanes 2 and 5) and 60 minutes (lanes 3 and 6) with plastic-bound monoclonal α-CD3 antibodies. The membrane on which the SDS-PAGE separated whole cell lysates were transferred, was first probed with an antibody specific for phosphorylated ERK-1/2, then, after stripping, it was reprobed with an α-ERK antibody (A). Alternatively, the membrane was first probed with an antibody specific for phosphorylated Raf, then, after stripping, it was reprobed with an α-Raf antibody (B). The membranes were also reprobed with an antibody specific for β-tubulin, in order to verify that equivalent amounts of proteins were loaded in each lane.

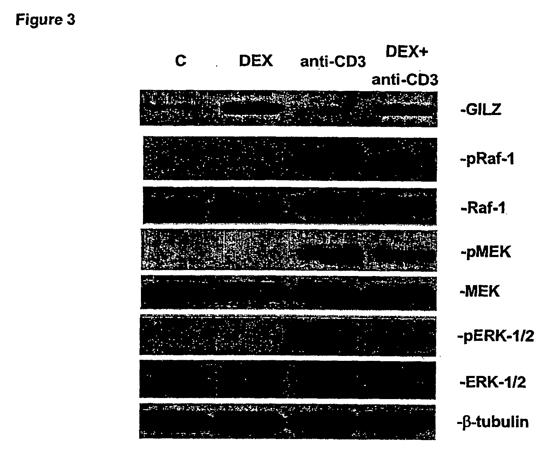

- Figure 3:: Western blot analysis showing the effect of DEX-induced GILZ over-expression on the phosphorylation of Raf, MEK, and ERK-1/-2. The membranes were stripped and reprobed as in figure 2.

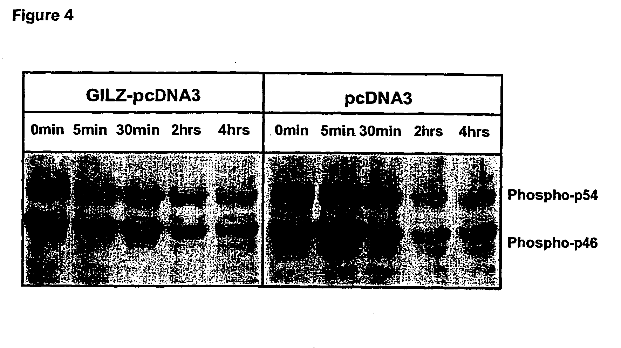

- Figure 4:: Western blot analysis showing the effect of GILZ over-expression on the phosphorylation pattern of SAPK/JNK. 3DO clones transfected with pcDNA3 (clone PV6) or GILZ-pcDNA3 (clone GIRL-19) were stimulated for the times indicated, with plastic-bound monoclonal α-CD3 antibodies. Whole cell lysates were probed with an antibody recognising both phosphorylated forms of JNK (phospho-p54, phospho-p46).

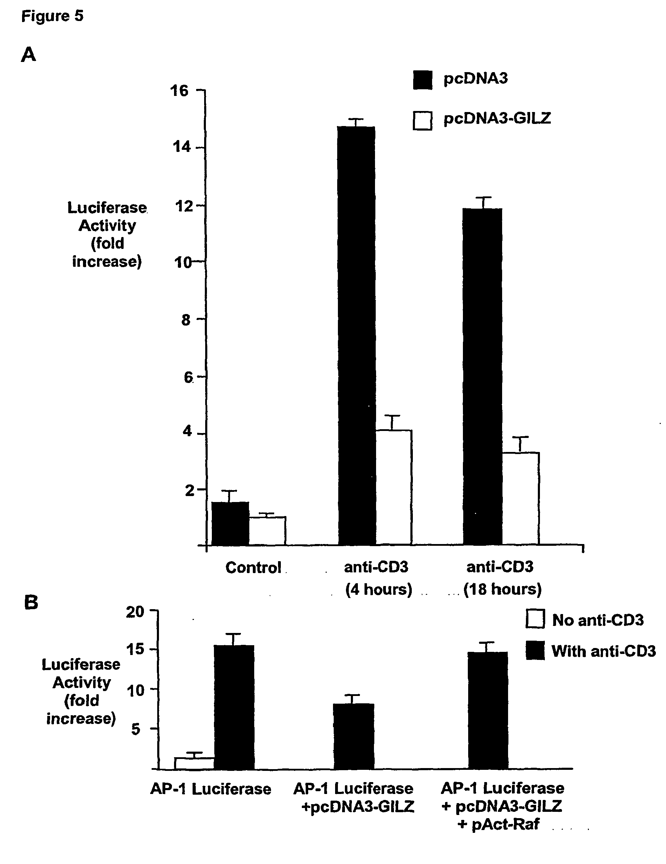

- Figure 5:: Luciferase assay using transiently transfected 3DO with a AP-1 controlled luciferase gene, un / stimutated with anti-CD3 antibodies in presence of different of plasmids. The stimulation was of 1 hour in (B).

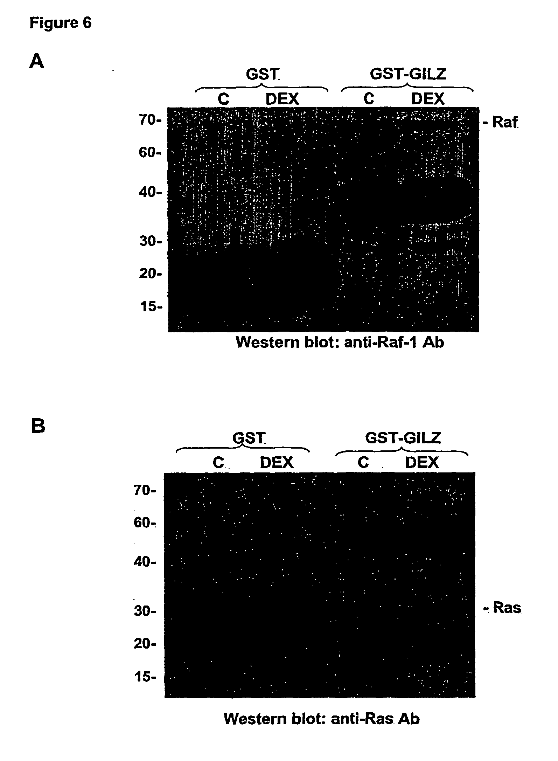

- Figure 6:: Western blot analysis demonstrating the protein-protein interaction between endogenous Raf / Ras and GST-GILZ. The GST pulldown assay was performed in the presence of 3DO whole cell extracts (500 micrograms), trated or untreated with DEX (100nM). The Western blot was peformed with the indicated primary antibodies.

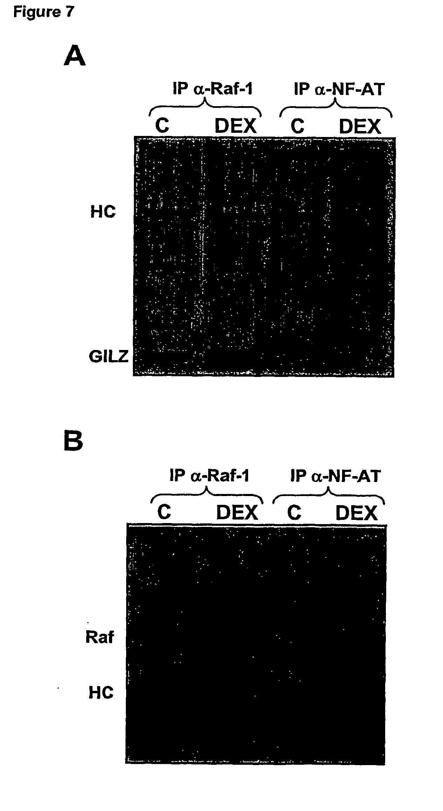

- Figure 7:: Mouse thymocytes were treated for 6 hours with DEX. Whole cell lysates were immunoprecipitated (IP) with an α-Raf or α-NF-AT (used as control) antibodies. The membranes were probed with an α-GILZ antiserum (A), stripped and reprobed with the α-Raf antibody (B). The position of immunoglobulins Heavy Chains is indicated with HC.

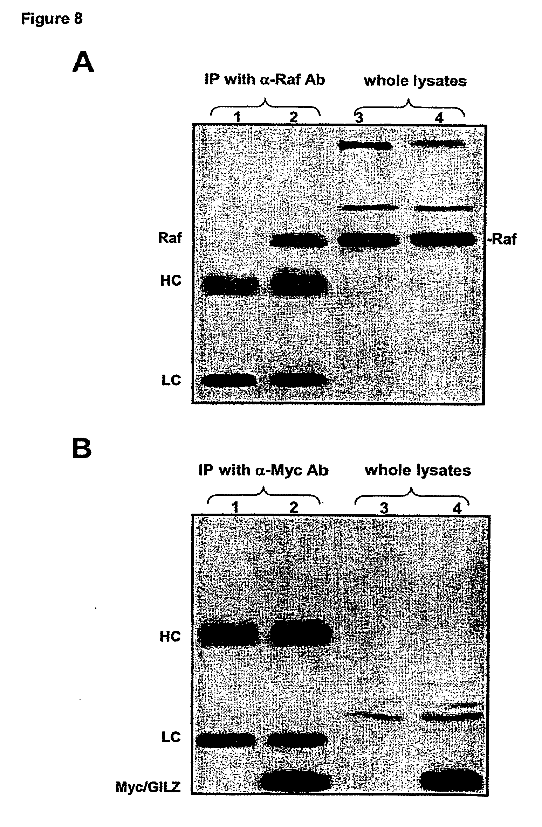

- Figure 8:: Western blot analysis demonstrating the interaction between GILZ and Raf in transfected cells. COS-7 cells were co-transfected with either only pUSEamp-Raf (an expression vector carrying Raf, lanes 1 and 3) or pUSEamp-Raf and pcDNA3.1/Myc-His-GILZ (an expression vector carrying Myc-tagged GILZ, lanes 2 and 4). The position of immunoglobulins Heavy and Light Chains are indicated with HC and LC, respectively.

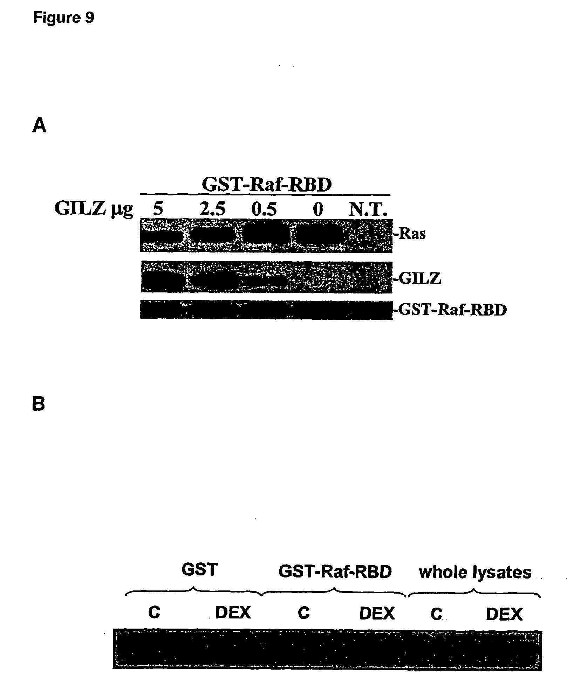

- Figure 9:: Western blot analysis demonstrating that the interaction between Raf and GILZ is mediated by the Raf region comprising the Ras binding domain. GST-pull-down assay was performed with GST-Raf-RBD, a fusion protein comprising the human Ras Binding Domain (RBD) or GST alone using. The whole cell lysates were obtained from COS-7 cell line overexpressing Ras (N. T. not transfected cells), then challenged with GST-Raf-RBD and different amount of GILZ (A), or were obtained from DEX-un/treated thymocytes (B). The membrane was probed with antibodies against the indicated antibodies. Total lysates from DEX-treated and untreated thymocytes were loaded to control GILZ expression.

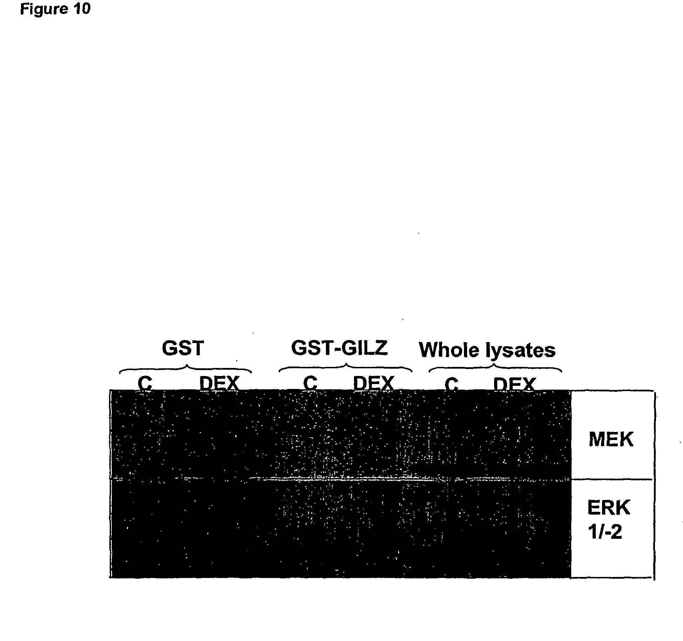

- Figure 10:: Western blot demonstrating the specificity of the interaction between GILZ and Raf. GST-pull-down experiments were performed with GST-GILZ fusion protein containing GILZ full-length protein or GST alone incubated in the presence of whole cell lysates. Western blot was performed with α-MEK or α-ERK antibodies.

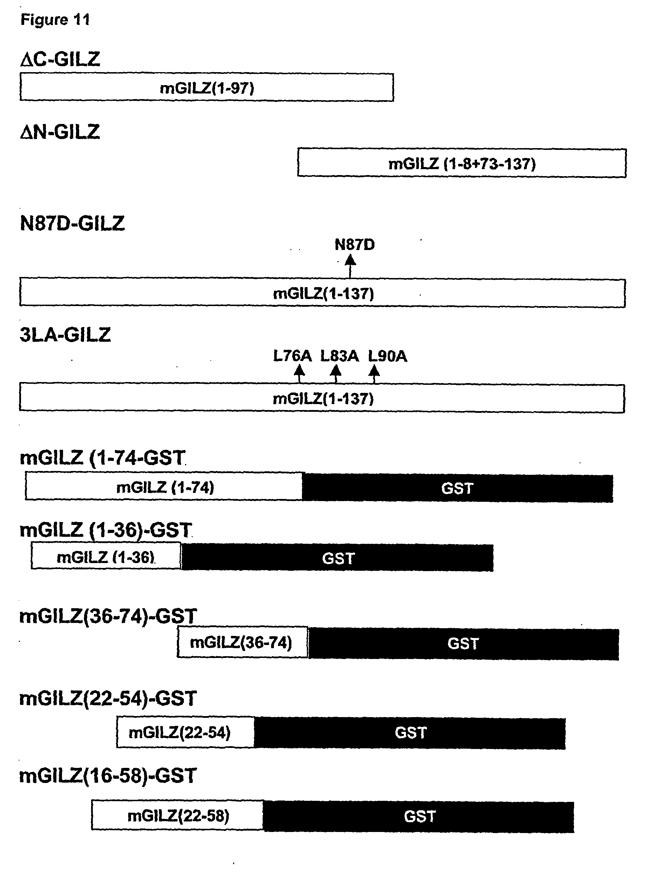

- Figure 11:: schematic representation of the GILZ mutants used in the pull-down experiments.

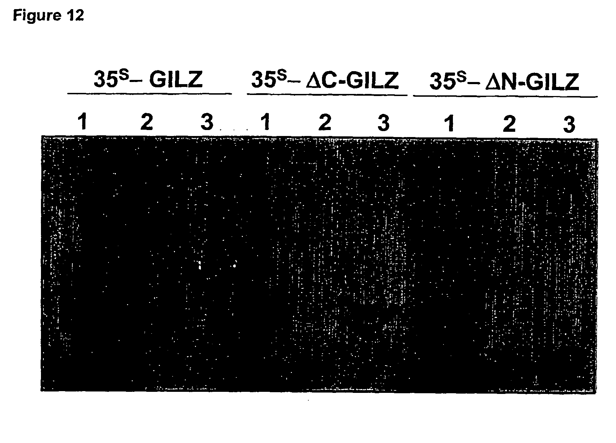

- Figure 12:: Western blot analysis demonstrating that the interaction between the Ras binding domain of Raf and GILZ is mediated by a domain located in the N-terminal region of GILZ. GST-Raf-RBD fusion protein attached to glutathione sepharose beads was incubated overnight with *in vitro* 35^{S}-labeled full GILZ (35^{S}-GILZ), or GILZ lacking the C-terminal region (35^{S}-ΔC-GILZ) or GILZ lacking the N-terminal region (35^{S}-ΔN-GILZ). Lane 1, sample of radiolabeled protein. Lane 2, radiolabeled protein immobilized on GST beads. Lane 3, radiolabeled protein immobilized on GST-Raf-RBD beads.

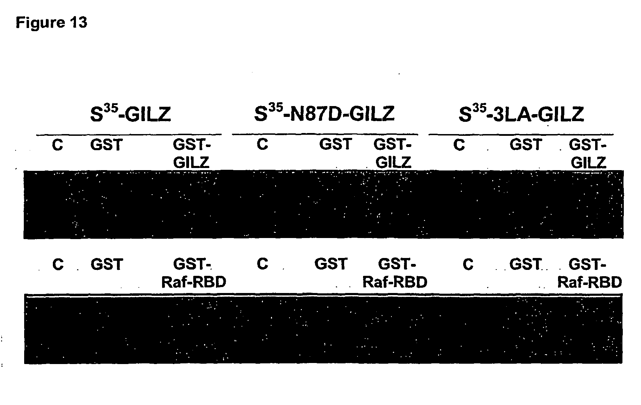

- Figure 13:: Western blot analysis demonstrating that the interaction between the Ras binding domain of Raf and GILZ is mediated by a GILZ domain distinct from the GILZ dimerization domain. GST-GILZ or GST-Raf-RBD fusion proteins immobilized onto glutathione sepharose beads were incubated overnight with *in vitro* 35^{S}-labeled full GILZ or GILZ mutants. Lane C, sample of radiolabeled protein.

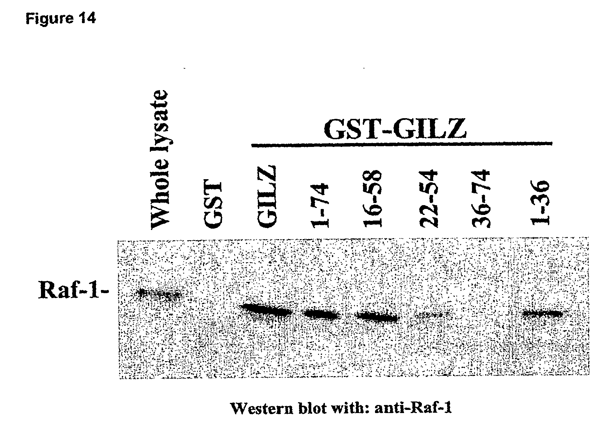

- Figure 14:: Western blot showing the different efficiency with which different GILZ fragments bind Raf in cell extracts in a GST pull down assay using 3DO cells (see figure 6).

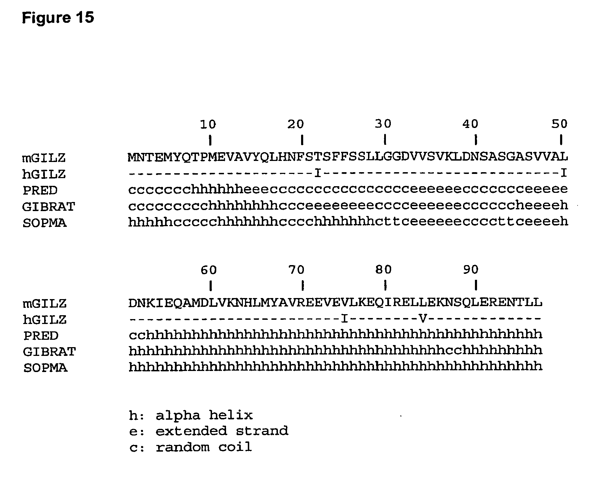

- Figure 15:: Secondary structure predictions for N-terminal regions of mouse GILZ (mGILZ) and human GILZ (hGILZ), corresponding to SEQ ID NO: 1 and SEQ ID NO: 2, respectively. Residues identical in mouse and human GILZ are indicated with -. The prediction were obtained by using the following methods: PREDATOR (PRED; Frishman D and Argos P, Protein Eng 1996, 9(2):133-142), GIBRAT (Gibrat, JF et al., J Mol Biol 1987, 198: 425-443), SOPMA (Geourjon C and Deléage G, Cabios 1995 11: 681-684). These methods can be available through different interfaces, for example at the NPS@ site (http://npsa-pbil.ibcp.fr/cgi-bin/npsa_automat.pl?page=/NPSA/npsa_server.html)

### DETAILED DESCRIPTION OF THE INVENTION

In view of the above mentioned evidences in the prior art, even though protein-protein interactions were known at the level of protein directly involved in transcription, there was no indication that GILZ, or any specific GILZ peptide, could directly interact with proteins controlling signal transduction pathways, modulating consequently the downstream cell activation.

By investigating the molecular mechanism responsible of the AP-1 activation driven by the Mitogen Activated Protein Kinases (MAPKs) cascade, it has now surprisingly found that GILZ can also affect the Raf/Ras complex-mediated intracellular signaling cascade by the means of a protein-protein interaction. This interaction could be responsible, at least in part, for GILZ-induced TCR unresponsiveness and, via inhibition of Raf-MEK-ERK activation pathway, for the regulation of the immune response mediated by GCHs.

Prior to the present invention, this finding was not known or predictable and provides novel compounds, novel compositions capable of inhibiting MAPKs pathway, screening assays, and therapeutic methods for treating diseases.

Accordingly, the present invention provides the use of GILZ protein for inhibiting the Mitogen Activated Protein Kinases (MAPKs) pathway in an organism, in an organ, in a tissue, or in cultured cells. Moreover, novel peptides capable of binding Raf protein and of inhibiting the MAPKs pathway are selected from:
a) peptides having the amino acid sequence SEQ ID NO: 3;
b) fragments of the N-terminal domain of GILZ comprising at least 5 consecutive amino acids of SEQ ID NO: 3;
c) active mutants of (a) or (b) in which one or more amino acid residues have been added, deleted, or substituted;
d) polypeptides or peptides comprising (a), (b), or (c), and an amino acid sequence belonging to a protein other than GILZ;

The amino acid sequence SEQ ID NO: 3 corresponds to the residues 16-36 of mouse GILZ, which has been shown to be the region of GILZ which binds Raf, mediating the inhibition of MAPKs cascade determined by GILZ, in the examples of the present patent application.

The prior art originally disclosing human and murine GILZ sequences (WO 98/49291; EP 884385 A1), as well as the prior art disclosing the interaction between c-Fos and c-Jun and the N-terminal residues 1-60 of GILZ (Mittelstadt PR and Ashwell JD, 2001) failed to demonstrate both the possibility and the effects of GILZ interaction with Raf/Ras complex and the interacting properties of specific GILZ regions smaller than the major protein domains.

In preferred embodiments, the polypeptides or peptides comprising at least 5 consecutive amino acids of SEQ ID NO: 3 are fragments of the N-terminal domain of GILZ corresponding to structural elements of such region, as well GILZ fragments including, partly or completely, sequences belonging to one or more of these structural elements. Examples of these peptides correspond to the sequences GILZ(1-20), GILZ(21-50), GILZ (1-50), GILZ (10-30), GILZ (10-40), GILZ (16-22), GILZ (30-36), GILZ (10-50), GILZ (30-50), GILZ(16-58), or GILZ(1-36).

Such fragments are essentially GILZ "analogs", that is, displaying substantially the same novel biological activity of GILZ characterized in the present invention, as determined by means of routine experimentation comprising subjecting such an analog to the assays disclosed in the Examples below. These analogs are prepared by known synthesis and/or by site-directed mutagenesis techniques, or any other known technique suitable thereof.

Deletions, substitutions, or additions in the above defined GILZ fragments can provide novel molecules which are active mutants of such fragments. Active mutants of the polypeptide or peptide as defined in the present invention, or nucleic acid coding therefore, include a finite set of substantially corresponding sequences as substitution peptides or polypeptides which can be routinely obtained by one of ordinary skill in the art, without undue experimentation, based on the teachings and functional features presented in the Examples. Nonetheless, they should display the same biological activity (i.e. inhibiton of Raf/Ras complex mediated signal transduction) as demonstrated in the present invention, or by any other relevant means known in the art, at comparable or higher levels.

In accordance with the present invention, preferred changes in these active mutants are commonly known as "conservative" or "safe" substitutions. Conservative amino acid substitutions are those with amino acids having sufficiently similar chemical properties, in order to preserve the structure and the biological function of the molecule. It is clear that insertions and deletions of amino acids may also be made in the above defined sequences without altering their function, particularly if the insertions or deletions only involve a few amino acids, e.g., under thirty, and preferably under ten, and do not remove or displace amino acids which are critical to the functional conformation of the relevant GILZ fragment.

The literature provide many models on which the selection of conservative amino acids substitutions can be performed on the basis of statistical and physico-chemical studies on the sequence and/or the structure of natural protein (Rogov Sl and Nekrasov AN, 2001). Protein design experiments have shown that the use of specific subsets of amino acids can produce foldable and active proteins, helping in the classification of amino acid substitutions which can be more easily accommodated in protein structure, and which can be used to detect functional and structural homologs and paralogs (Murphy LR et al., 2000). Preferably, the synonymous amino acid groups and more preferred synonymous groups are those defined in Table I.

Alternatively, specific active mutants may be designed for improving certain properties independent from Raf/Ras interaction. For example, active mutants may result from the introduction of metal binding site(s) for improving protein stability, without loss of function, in particular by replacing surface residues in a loop / turn region with histidine capable of binding His-metal ligands, such as nickel cation (Bell AJ Jr et al., 2002).

"Identity", as used herein, refers to the subunit sequence similarity between two polymeric molecules, e.g., two peptides. When a subunit position in both of the two molecules is occupied by the same monomeric unit, e.g., if a position in each of two peptides is occupied by Serine, then they are identical at that position. The identity between two sequences is a direct function of the number of matching or identical positions, e.g., if identical; if 90% of the positions, e.g., 9 of 10, are matched, the two sequences share 90% sequence identity.

The term "polypeptide" is used herein as a generic term to refer to native or recombinant proteins, fragments, or analogs of a polypeptide sequence. Thus, native or recombinant proteins, fragments, and analogs are species of the polypeptide group.

The term "peptide" will ordinarily applied to a polypeptidic chain containing from 4 to 80 or more contiguous amino acids, usually about 4-20 contiguous amino acids. Such peptides can be generated by methods known to those skilled in the art, including partial proteolytic cleavage of the protein, chemical synthesis of the fragment, or genetic engineering.

The term "fragment" as used herein refers to a polypeptide that has an N-terminal and/or C-terminal deletion when compared to the parent sequence (i.e. GILZ N-terminal region), but where the remaining amino acid sequence is identical to the corresponding positions in the naturally occurring sequence deduced, for example, from a full length cDNA sequence. Fragments typically contain at least 10 amino acids, preferably at least 20 amino acids or more.

The term "active" means that such alternative compounds should maintain the functional features characterized for GILZ and the specific fragment GILZ (18-36), accordingly to the present invention, and should be as well pharmaceutically acceptable, i.e. without imparting toxicity to the pharmaceutical compositions containing them.

In other preferred embodiments, the polypeptides or peptides of the invention comprise the above defined GILZ fragments or their active mutants, and an amino acid sequence belonging to a protein other than GILZ;). In still preferred embodiments, the peptides contain 4 to 25 amino acids, and have at least 70, 80, or 90% sequence identity to SEQ ID NO: 3.

The previous embodiments include, amongst the compounds of the invention, the amino acid sequence of other proteins belonging to the TSC-22/DIP family expressed by different organisms. An example is human GILZ which differs from mouse GILZ only for one residue in position 22, a (Threonine instead of an Isoleucine; figure 12).

The present definition of the compounds of the invention comprises also the corresponding "fusion proteins", i.e. polypeptides comprising the amino acid sequence SEQ ID NO: 3, or fragments and active mutants thereof, and an amino acid sequence belonging to a protein other than any GILZ-like protein. This latter sequence may provide additional properties without impairing considerably functional binding and inhibiting activities.

Examples of such additional properties are an easier purification procedure, a longer lasting half-life in body fluids, an additional binding moiety, the maturation by means of an endoproteolytic digestion, or intracellular localization. This latter feature is of particular importance for defining a specific group of fusion or chimeric proteins included in the above definition since it allows the molecules defined as inhibitors of Ras/raf complex mediated signal transduction in this patent application to be localized in the space where it should interact with Ras /Raf. Design of the moieties, ligands, and linkers, as well methods and strategies for the construction, purification, detection and use of fusion proteins are widely discussed in the literature (Nilsson J et al., 1997; "Applications of chimeric genes and hybrid proteins" Methods Enzymol. Vol. 326-328, Academic Press, 2000; WO 01/77137). The choice of one or more of these additional sequences to be fused to the GILZ-derived peptides of the invention is functional to the specific use and/or preparation method.

The polypeptides and the peptides of the present invention can provided in other alternative forms which can be preferred according to the desired method of use and/or production, for example as active fractions, precursors, salts, or derivatives.

The term "fraction" refers to any fragment of the polypeptidic chain of the compound itself, alone or in combination with related molecules or residues bound to it, for example residues of sugars or phosphates, or aggregates of the original polypeptide or peptide. Such molecules can result also from other modifications which do not normally alter primary sequence, for example *in vivo* or *in vitro* chemical derivatization of peptides (acetylation or carboxylation), those made by modifying the pattern of glycosylation (by exposing the peptide to enzymes which affect glycosylation e.g., mammalian glycosylating or deglycosylating enzymes) or phosphorylation (introduction of phosphotyrosine, phosphoserine, or phosphothreonine residues) of a peptide during its synthesis and processing or in further processing steps. In particular, the nature, the effect and the distribution of protein glycosylation have been reviewed in the literature (, 2002; Thanka Christlet TH and Veluraja K, 2001; Imperiali B and O'Connor SE, 1999).

The "precursors" are compounds which can be converted into the compounds of present invention by metabolic and enzymatic processing prior or after the administration to the cells or to the body.

The term "salts" herein refers to both salts of carboxyl groups and to acid addition salts of amino groups of the peptides, polypeptides, or analogs thereof, of the present invention. Salts of a carboxyl group may be formed by means known in the art and include inorganic salts, for example, sodium, calcium, ammonium, ferric or zinc salts, and the like, and salts with organic bases as those formed, for example, with amines, such as triethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid. Of course, any such salts must have substantially similar activity to the peptides, polypeptides of the invention or its analogs.

The term "derivatives" as herein used refers to derivatives which can be prepared from the functional groups present on the lateral chains of the amino acid moieties or on the terminal N- or C- groups according to known methods. Such derivatives include for example esters or aliphatic amides of the carboxyl-groups and N-acyl derivatives of free amino groups or O-acyl derivatives of free hydroxyl-groups and are formed with acyl-groups as for example alcanoyl- or aroyl-groups. Alternatively, useful conjugates or complexes of the antagonists of the present invention can be generated as derivatives, using molecules and methods known in the art for improving the detection of the interaction with other proteins (radioactive or fluorescent labels, biotin), therapeutic efficacy (cytotoxic agents, isotopes), or drug delivery efficacy, such as polyethylene glycol and other natural or synthetic polymers (Pillai O and Panchagnula R, 2001). In the latter case, the antagonists may be produced following a site-directed modification of an appropriate residue, present in the natural sequence or introduced by mutating the natural sequence, at an internal or terminal position. Similar modifications have been already disclosed for small polypeptides such as chemokines (WO 02/04499; WO 02/04015; Vita C et al., 2002).

Any residue can be used for attachment, provided it has a side-chain amenable for polymer attachment (i.e., the side chain of an amino acid bearing a functional group, e.g., lysine, aspartic acid, glutamic acid, cysteine, histidine, etc.). Alternatively, a residue at these sites can be replaced with a different amino acid having a side chain amenable for polymer attachment. Also, the side chains of the genetically encoded amino acids can be chemically modified for polymer attachment, or unnatural amino acids with appropriate side chain functional groups can be employed. Polymer attachment may be not only to the side chain of the amino acid naturally occurring in a specific position of the antagonist or to the side chain of a natural or unnatural amino acid that replaces the amino acid naturally occurring in a specific position of the antagonist, but also to a carbohydrate or other moiety that is attached to the side chain of the amino acid at the target position.

Polymers suitable for these purposes are biocompatible, namely, they are nontoxic to biological systems, and many such polymers are known. Such polymers may be hydrophobic or hydrophilic in nature, biodegradable, non-biodegradable, or a combination thereof. These polymers include natural polymers (such as collagen, gelatin, cellulose, hyaluronic acid), as well as synthetic polymers (such as polyesters, polyorthoesters, polyanhydrides). Examples of hydrophobic non-degradable polymers include polydimethyl siloxanes, polyurethanes, polytetrafluoroethylenes, polyethylenes, polyvinyl chlorides, and polymethyl methaerylates. Examples of hydrophilic non-degradable polymers include poly(2-hydroxyethyl methacrylate), polyvinyl alcohol, poly(N-vinyl pyrrolidone), polyalkylenes, polyacrylamide, and copolymers thereof. Preferred polymers comprise as a sequential repeat unit ethylene oxide, such as polyethylene glycol (PEG).

The preferred method of attachment employs a combination of peptide synthesis and chemical ligation. Advantageously, the attachment of a water-soluble polymer will be through a biodegradable linker, especially at the amino-terminal region of a protein. Such modification acts to provide the protein in a "pro-drug" form, that, upon degradation of the linker releases the protein without polymer modification.

The above described alternative compounds term are intended to comprehend molecules with changes to the sequence of the GILZ protein which do not affect the basic characteristics disclosed in the present patent application, particularly insofar as its ability of binding Raf/Ras and inhibiting MAPKs pathway is concerned. Similar changes are generally considered to provide compounds having an activity essentially corresponding to the one of GILZ, and may result from conventional mutagenesis techniques of the encoding DNA, from combinatorial technologies at the level of encoding DNA /protein sequence (such as DNA shuffling, phage display/selection), or from computer-aided design studies, followed by the screening for the desired activity as described in the Examples below.

In particular, the invention includes alternative molecules based on GILZ-derived peptides which are generated in the form of peptide mimetics (also called peptidomimetics), that is, GILZ analogs in which the nature of peptide or polypeptide has been chemically modified at the level of amino acid side chains, of amino acid chirality, and/or of the peptide backbone. These alterations are intended to provide GILZ agonist compounds having similar or improved therapeutic and/or pharmacokinetic properties.

For example, when the peptide is susceptible to cleavage by peptidases following injection into the subject is a problem, replacement of a particularly sensitive peptide bond with a non-cleavable peptide mimetic can provide a peptide more stable and thus more useful as a therapeutic. Similarly, the replacement of an L-amino acid residue is a standard way of rendering the peptide less sensitive to proteolysis, and finally more similar to organic compounds other than peptides. Also useful are amino-terminal blocking groups such as t-butyloxycarbonyl, acetyl, theyl, succinyl, methoxysuccinyl, suberyl, adipyl, azelayl, dansyl, benzyloxycarbonyl, fluorenylmethoxycarbonyl, methoxyazelayl, methoxyadipyl, methoxysuberyl, and 2,4,-dinitrophenyl.

Many other modifications providing increased potency, prolonged activity, easiness of purification, and/or increased half-life have been described in the literature (WO 02/10195; Villain M et al., 2001).In particular, blocking the charged N- and C-termini of the peptides would have the additional benefit of enhancing passage of the peptide through the hydrophobic cellular membrane and into the cell. Preferred alternative groups for amino acids included in peptide mimetics are those defined in Table II.

The techniques for the synthesis and the development of peptide mimetics and other non-peptide mimetics are well known in the art (Hruby VJ and Balse PM, 2000; Golebiowski A et al., 2001; Kim HO and Kahn M, 2000). For example, miniproteins and synthetic mimics able of disrupting protein-protein interactions and inhibiting protein complex formation have been described (Cochran AG, 2001). Various methodology, for incorporating unnatural amino acids into proteins, using both *in vitro* and *in vivo* translation systems, to probe and/or improve protein structure and function are also disclosed in the literature (Dougherty DA, 2000).

Methods for optimising the peptide structure of series of peptides derived from a scaffold, following a computational stabilization and/or optimization of proteins, can be developed using cell-/ peptide-based microarrays or other experimental screening technologies (WO 02/90985; Wu RZ et al., 2002; Filikov AV et al., 2002; Hayes RJ et al., 2002).

Compounds of the invention having MAPKs cascade inhibiting properties and having as well some lipophilic characteristics may be most useful in view of the fact that in practice, such compounds to be used pharmaceutically should have the ability to pass through the cell membrane. Alternatively, such compounds can be chemically modified (i.e. derivatized, conjugated or complexed) with molecules that, being transported naturally across the cell membrane, facilitate their entry or enhance their permeability across the cell membrane and into the cytoplasm. Examples of these membrane blending agents are fusogenic polypeptides, ion-channel forming polypeptides, other membrane polypeptides, and long chain fatty acids, e.g., myristic acid, palmitic acid (US 5149782). These membranes blending agents insert the molecular conjugates into the lipid bilayer of cellular membranes and facilitate their entry into the cytoplasm. Other valuable methods for transmembrane delivery of molecules exploit the mechanism of receptor mediated endocytotic activity. These receptor systems include those recognizing galactose, mannose, mannose 6-phosphate, transferrin, asialoglycoprotein, transcobalamin (vitamin B 12), insulin and other peptide growth factors such as epidermal growth factor (EGF). Nutrient receptors, such as receptors for biotin and folate, can be also advantageously used to enhance transport across the cell membrane due to the location and multiplicity of biotin and folate receptors on the membrane surfaces of most cells and the associated receptor mediated transmembrane transport processes (US 5108921). Thus, a complex formed between a compound to be delivered into the cytoplasm and a ligand, such as biotin or folate, can be contacted with a cell membrane bearing biotin or folate receptors to initiate the receptor mediated trans-membrane transport mechanism and thereby permit entry of the desired compound into the cell. Specific examples for intracellular delivery of Ras/Raf interacting peptides are provided in the literature (Maruta H et al., 2002).

Modifications of the compounds of the invention to improve penetration of the blood-brain barrier would also be useful. Peptides may be altered to increase lipophilicity (e.g. by esterification to a bulky lipophilic moiety such as cholesteryl) or to supply a cleavable "targetor" moiety that enhances retention on the brain side of the barrier (Bodor et al., 1992). Alternatively, the peptide may be linked to an antibody specific for the transferrin receptor, in order to exploit that receptor's role in transporting iron across the blood- brain barrier (Friden et al., Science 1993, 259: 373-377). Other methods of biomimetic transport and rational drug delivery in the field of transvascular drug delivery are known in the art (Ranney DF, Biochem Pharmacol 2000, 59: 105-14).

The compounds of the invention may be prepared by any well known procedure in the art, including recombinant DNA-related technologies or chemical synthesis technologies. The expression of peptides and polypeptides of the invention can be achieved in an Eukaryotic or Prokaryotic cell by introducing an expression vector that, either integrated in the genome of the cell or maintained as an episome, contains the nucleotide sequence coding for the desired polypeptide or peptide under the control of transcriptional initiation/termination regulatory sequences which are constitutively active or inducible in said cell. Alternatively, the relevant coding sequence may be already present in the genomic DNA of the cell and its expression can be activated by introducing exogenous regulatory sequences, as described in the prior art (EP505500).

The DNA sequences coding for the GILZ-derived peptides and proteins of the invention can be isolated from the corresponding human or mouse genomic DNA or cDNA sequences, or any other nucleic acid sequences which, by virtue of the degeneracy of the genetic code, also encodes for the given amino acid sequences. Expression vectors which comprise the above DNAs, together with any appropriate stop / start trascription and translation elements and any other additional sequence (e.g. heterologus sequence to be included in a fusion protein) can be used to transform host cells which can be cultured in an appropriate culture media, before collecting the expressed proteins and further processing.

Expression of any of the recombinant proteins of the invention as mentioned herein can be effected in Eukaryotic cells (e.g. yeasts, insect or mammalian cells) or Prokaryotic cells, using the appropriate expression vectors. Any method known in the art can be employed. The coding sequences can be accordingly chosen in order to have an optimal codon usage for expression according to the specific the host cell, for example *E. coli* (Kane JF et al., 1995). Recombinant proteins having the desired glycosylation pattern can be obtained by selecting the appropriate mammalian host cells (Grabenhorst E et al., 1999).

In particular, mammalian cells, such as human, monkey, mouse, and Chinese hamster ovary (CHO) cells in particular, are preferred because they provide post-translational modifications to protein molecules, including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e., pre-peptides, signal sequences).

Factors of importance in selecting a particular plasmid or viral vector include: the ease with which recipient cells that contain the vector, may be recognized and selected from those recipient cells which do not contain the vector; the number of copies of the vector which are desired in a particular host; and whether it is desirable to be able to "shuttle" the vector between host cells of different species.

The vectors should allow the expression of the isolated or fusion protein including the antagonist of the invention in the Prokaryotic or Eukaryotic host cell under the control of transcriptional initiation / termination regulatory sequences, which are chosen to be constitutively active or inducible in said cell. After the introduction of the vector(s), the host cells are grown in a selective medium, which selects for the growth of vector-containing cells. Expression of the cloned gene sequence(s) results in the production of the desired proteins. A cell line substantially enriched in such cells can be then isolated to provide a stable cell line.

For Eukaryotic hosts (e.g. yeasts, insect or mammalian cells), different transcriptional and translational regulatory sequences may be employed, depending on the nature of the host. They may be derived form viral sources, such as adenovirus, bovine papilloma virus, Simian virus or the like, where the regulatory signals are associated with a particular gene which has a high level of expression. Examples are the TK promoter of the Herpes virus, the SV40 early promoter, the yeast ga14 gene promoter, etc. Transcriptional initiation regulatory signals may be selected which allow for repression and activation, so that expression of the genes can be modulated. The cells which have been stably transformed by the introduced DNA can be selected by also introducing one or more markers which allow for selection of host cells which contain the expression vector. The marker may also provide for phototrophy to an auxotropic host, biocide resistance, e.g. antibiotics, or heavy metals such as copper, or the like. The selectable marker gene can either be directly linked to the DNA gene sequences to be expressed, or introduced into the same cell by co-transfection.

These objects of the invention can be achieved by combining the disclosure provided by the present patent application on GILZ-derived peptides, with the knowledge of common molecular biology techniques. Many books and reviews provides teachings on how to clone and produce recombinant proteins using vectors and Prokaryotic or Eukaryotic host cells, such as some titles in the series "A Practical Approach" published by Oxford University Press ("DNA Cloning 2: Expression Systems", 1995; "DNA Cloning 4: Mammalian Systems", 1996; "Protein Expression", 1999; "Protein Purification Techniques", 2001).

The GILZ-derived peptides and proteins of the invention may be prepared by any other well known procedure in the art, in particular, by the chemical synthesis procedures, which can be efficiently applied on these molecule given the short length. Even totally synthetic proteins, also containing additional chemical groups, are disclosed in the literature (Brown A et al., 1996; Vita C et al., 2002).

Examples of chemical synthesis technologies are solid phase synthesis and liquid phase synthesis. As a solid phase synthesis, for example, the amino acid corresponding to the C-terminus of the peptide to be synthesised is bound to a support which is insoluble in organic solvents, and by alternate repetition of reactions, one wherein amino acids with their -amino groups and side chain functional groups protected with appropriate protective groups are condensed one by one in order from the C-terminus to the N-terminus, and one where the amino acids bound to the resin or the protective group of the -amino groups of the peptides are released, the peptide chain is thus extended in this manner. Solid phase synthesis methods are largely classified by the tBoc method and the Fmoc method, depending on the type of protective group used. Typically used protective groups include tBoc (t-butoxycarbonyl), CI-Z (2-chlorobenzyloxycarbonyl), Br-Z (2-bromobenzyloxycarbonyl), Bzl (benzyl), Fmoc (9-fluorenylmethoxycarbonyl), Mbh (4,4'-dimethoxydibenzhydryl), Mtr (4-methoxy-2,3,6-trimethylbenzenesulphonyl), Trt (trityl), Tos (tosyl), Z (benzyloxycarbonyl) and Cl2-Bzl (2,6-dichlorobenzyl) for the amino groups; NO2 (nitro) and Pmc (2,2,5,7,8-pentamethylchromane-6-sulphonyl) for the guanidino groups); and tBu (t-butyl) for the hydroxyl groups). After synthesis of the desired peptide, it is subjected to the de-protection reaction and cut out from the solid support. Such peptide cutting reaction may be carried with hydrogen fluoride or trifluoromethane sulfonic acid for the Boc method, and with TFA for the Fmoc method.

Purification of the synthetic or recombinant proteins may be carried out by any one of the methods known for this purpose, i.e. any conventional procedure involving extraction, precipitation, chromatography, electrophoresis, or the like. For example, HPLC (high performance liquid chromatography) can be used. The elution can be carried using a water-acetonitrile-based solvent commonly employed for protein purification. A further purification procedure that may be used in preference for purifying the peptides or proteins of the invention is affinity chromatography using monoclonal antibodies, heparin, or any other suitable ligand which can bind the target protein at high efficiency and can be immobilized on a gel matrix contained within a column. Impure preparations containing the proteins are passed through the column. The protein will be bound to the column by means of this ligand while the impurities will pass through. After washing, the protein is eluted from the gel by a change in pH or ionic strength.

The invention includes purified preparations of the compounds of the invention. Purified preparations, as used herein, refers to the preparations which contain at least 1 %, preferably at least 5%, by dry weight of the compounds of the invention.

The GILZ-derived peptides and proteins of the present invention can be also used in methods and kits for screening *in vitro* and *in vivo* compounds possibly binding to Raf and/or Ras and inhibiting MAPKs pathway, as provided in the Examples of the present patent application, by comparing the effect of such compounds with the effect provided by the GILZ-derived peptides and proteins of the invention. Using methods known in the art, the components of this screening assay can be immobilized (by adsorption onto a plastic microtiter plate or specific binding of a fusion protein to a polymeric bead containing an affinity group), co-precipitated (by antibodies), and/or can be labeled (using radioisotopes, enzymatic labels, fluorescers, chemiluminescers).

Accordingly, the present invention also provides compounds isolated, identified and/or characterized by any of the above in vivo or in vitro assays and exemplified in the present patent application, as well any other chemical compound identified by methods of computer-aided drug design which make use of the sequence and structure information related to Raf/Ras and GILZ, in particular of the respective binding surfaces, to derive peptides or other organic compounds to be synthetized and tested *in vitro* and *in vivo* as inhibitors of MAPKs pathway.

Quantitative structure-activity investigations, which correlated structure-guided biochemical analysis with biological function of protein-protein interactions, have been performed on the basis of the tridimensional structure of Ras and Raf (Block C et al., 1996). Since the tridimensional structure of a fragment of a GILZ-like protein is known (Seidel G et al., 1997) and can thus be used to extract relevant information on the conformation of residues which are critical for binding, and consequently to derive chemical structures which can simulate the interaction site of GILZ with Raf/Ras. Such technologies of computational protein design and structure based drug design allow to identify and engineer proteins and other type of molecules that fold, signal, or adopt conformational states faster and with more efficacy, with a significant impact on biotechnology and chemical biology (Kraemer-Pecore CM et al., 2001; Sawyer TK, "Peptidomimetic and Nonpeptide Drug Discovery: impact of Structure-Based Drug Design" in "Structure Based Drug Design", edited by Veerapandian P, Marcel Dekker Inc., 1997, pg. 557-663).

Methods and software allowing protein structure homology/threading modeling, minimization, and docking are well known in the art, such as Insight II (MSI), 3D-PSSM (Kelley LA et al., 2000), and FTDOCK (Gabb HA et al., 1997). The results of these simulations can be later challenged once that a structure of a GILZ-derived and Raf/Ras-derived peptides complex is actually resolved by Nuclear Magnetic Resonance (NMR) spectroscopy or X-ray crystallography. Such inhibitory peptides can also be characterized by physical and chemical techniques (for example circular dichroism, fluorescence, electron spin resonance) that yield data concerning the local environment of the interacting peptides. Synthetic chemistry techniques can then be used as described above to produce compounds which mimic the inhibitory conformation of each peptide.

In vitro and/or computer assisted screening directed at small peptides (for example having between 4 and 25 amino acid) derived from GILZ N-terminal sequence, is advantageous to isolate and develop more stable peptide or peptidomimetic-type drugs. Once that these compounds have been screened and found to be capable of binding to Raf/Ras, the MAPKs inhibiting properties will then be assessed to demonstrate their expected utility.

Another aspect of the invention are methods for inhibiting unwanted cell proliferation mediated by the MAPKs in an animal, in an organ, in a tissue, or in cultured cells by administering an effective amount of a GILZ-derived compound of the invention. These molecules can inhibit the cellular mechanisms triggered by the phosphorylation of Raf and Erk-1/-2, which play a crucial role in the transduction of signal from the cell membrane and cytoplasmatic receptors towards the transcriptional machinery in the nucleus. The GILZ interaction provides the inhibition of the Raf/Ras-mediated intracellular signaling. Such inhibition is desirable in the treatment of unwanted cell proliferation, in general, and cancer, in particular.

Many findings indicate that Raf is a direct major effector of Ras function. The requirement of Raf activity for Ras effector signalling allows the compounds of the present invention to interrupt the Ras protein pathway of oncogenic activation in tumor cells (Kloog Y and Cox AD, 2000; Weinstein-Oppenheimer CR et al., 2000). The present invention provides a novel opportunity for the development of anticancer drugs targeting the MAP kinase pathway and controlling aberrant patterns of differentiation and proliferation (Sebolt-Leopold JS, 2000)

The interruption of the Raf/Ras-controlled MAPKs signaling cascade is expected to have antitumour activity in at least a proportion of human tumors (carcinomas, hematopoietic tumors of lymphoid/myeloid lineage, tumors of mesenchymal origin, melanoma). Therefore, the invention also relates to methods of manufacturing the compounds and pharmaceutical compositions, and methods of treating autoimmune or inflammatory diseases or cancers (such as lymphomas or lymphocytic leukaemia), which are triggered by Raf/Ras complex mediated activation. These compounds, as well as GILZ, can be used for the manufacture of a pharmaceutical composition for the treatment of autoimmune or inflammatory diseases or cancers. In such treatments, it can be sometimes advantageous to target the compounds of the present invention to the cancerous cells with the higher precision and specificity. Such targeting is well known within the art of cancer treatment and the preparation of suitable formulations and methods requires no more than routine experimentation.

Since blocking the Ras/Raf activation interferes with receptor-mediated activation of immune cells, this method may also be useful in downregulating the immune response in patients with inflammatory or autoimmune diseases such as systemic lupus erythematosus (SLE), type 1 diabetes, vasculitis, autoimmune chronic active hepatitis, ulcerative cholitiss, Crohn's disease, allergic diseases, nephritic syndrome, sarcoidosis, and rheumatoid arthritis. Suppression of an immune response using this method may also be useful in the treatment of allograft or xenograft recipients to prevent rejection of a transplanted organ.

The therapeutic administration of a peptide intracellularly can also be accomplished using gene therapy, wherein a nucleic acid which includes a promoter operatively linked to a sequence encoding an heterologous polypeptide or peptide is used to generate high levels of expression in cells transfected with the previously described nucleic acid. Plasmid DNA or isolated nucleic acid encoding GILZ-derived peptides or proteins of the invention may be introduced into cells of the patient by standard vectors and/or gene delivery systems. Suitable gene delivery systems may include liposomes, receptor-mediated delivery systems, naked DNA, and viral vectors such as herpes viruses, retroviruses, and adenoviruses, among others.

The compounds of the invention described above (proteins, peptides, organic compounds, etc.) may thus be used as medicaments, in particular as the active ingredients in pharmaceutical compositions for the treatment of unwanted cell proliferation, in general, and cancer in particular. Such treatments can be performed either *in vivo,* by administering the compound to the animal, or *ex vivo,* that is, the compounds are administered to an organ, a tissue, or cultured cells which have been extracted from the body and kept outside for a short period to provide a specific therapeutic treatment before being implanted again in the body.

The present invention also provides pharmaceutical compositions comprising one of the compounds of the invention, as active ingredient and a pharmaceutically acceptable carrier, excipient, stabilizer or diluent. The composition or the isolated compounds of the invention can be administered alone or in combination with a another composition or compound which provide additional beneficial effects by acting in a synergic or in a coordinated manner.

Pharmaceutical compositions comprising the MAPKs inhibitory compounds of the present invention include all compositions wherein said compound is contained in therapeutically effective amount, that is, an amount effective affect the course and the severity of the disease, leading to the reduction or remission of such pathology. The effective amount will depend on the route of administration and the condition of the patient.

The pharmaceutical compositions may contain suitable pharmaceutically acceptable carriers, biologically compatible vehicles which are suitable for administration to an animal (for example, physiological saline) and eventually comprising auxiliaries (like excipients, stabilizers or diluents) which facilitate the processing of the active compounds into preparations which can be used pharmaceutically.

The pharmaceutical compositions may be formulated in any acceptable way to meet the needs of the mode of administration. The use of biomaterials and other polymers for drug delivery, as well the different techniques and models to validate a specific mode of administration, are disclosed in literature (Luo B and Prestwich GD, 2001; Cleland JL et al., 2001).

Any accepted mode of administration can be used and determined by those skilled in the art. For example, administration may be by various parenteral routes such as subcutaneous, intravenous, intradermal, intramuscular, intraperitoneal, intranasal, transdermal, oral, or buccal routes. Parenteral administration can be by bolus injection or by gradual perfusion over time. Preparations for parenteral administration include sterile aqueous or non-aqueous solutions, suspensions, and emulsions, which may contain auxiliary agents or excipients which are known in the art, and can be prepared according to routine methods. In addition, suspension of the active compounds as appropriate oily injection suspensions may be administered. Suitable lipophilic solvents or vehicles include fatty oils, for example, sesame oil, or synthetic fatty acid esters, for example, sesame oil, or synthetic fatty acid esters, for example, ethyl oleate or triglycerides. Aqueous injection suspensions that may contain substances which increase the viscosity of the suspension include, for example, sodium carboxymethyl cellulose, sorbitol, and/or dextran. Optionally, the suspension may also contain stabilizers. Pharmaceutical compositions include suitable solutions for administration by injection, and contain from about 0.01 to 99 percent, preferably from about 20 to 75 percent of active compound together with the excipient. Compositions which can be administered rectally include suppositories.

It is understood that the dosage administered will be dependent upon the age, sex, health, and weight of the recipient, kind of concurrent treatment, if any, frequency of treatment, and the nature of the effect desired. The dosage will be tailored to the individual subject, as is understood and determinable by one of skill in the art. The total dose required for each treatment may be administered by multiple doses or in a single dose. The pharmaceutical composition of the present invention may be administered alone or in conjunction with other therapeutics directed to the condition, or directed to other symptoms of the condition.

The compounds of the present invention may be administered to the patient intravenously in a pharmaceutically acceptable carrier such as physiological saline. Standard methods for intracellular delivery of peptides can be used, e.g. delivery via liposomes. Such methods are well known to those of ordinary skill in the art. The formulations of this invention are useful for parenteral administration, such as intravenous, subcutaneous, intramuscular, and intraperitoneal.

As well known in the medical arts, dosages for any one patient depends upon many factors, including the patient's size, body surface area, age, the particular compound to be administered, sex, time and route of administration, general health, and other drugs being administered concurrently. Usually a daily dosage of active ingredient can be about 0.01 to 100 milligrams per kilogram of body weight. Ordinarily 1 to 40 milligrams per kilogram per day given in divided doses or in sustained release form is effective to obtain the desired results. Second or subsequent administrations can be performed at a dosage, which is the same, less than, or greater than the initial or previous dose administered to the individual.

The present invention has been described with reference to the specific embodiments, but the content of the description comprises all modifications and substitutions, which can be brought by a person skilled in the art without extending beyond the meaning and purpose of the claims. All references cited herein are entirely incorporated by reference herein, including all data, tables, figures, and text presented in the cited references. Additionally, the entire contents of the references cited within the references cited herein are also entirely incorporated by reference. Reference to known method steps, conventional method steps, known methods or conventional methods is not in any way an admission that any aspect, description or embodiment of the present invention is disclosed, taught or suggested in the relevant art. Unless defined otherwise, all technical and scientific terms used herein have the same meaning as commonly understood by one of ordinary skill in the art to which this invention belongs. Once understood the features of the methods and products disclosed in present application, the necessity and kind of additional steps can be easily deduced by reviewing prior art, as well as the non-limiting following figures and examples describing the basic details and some applications of the invention.

### EXAMPLES

### Example 1: effects of GILZ on the Raf-controlled MAPKs transduction pathway.

### Methods

### Cell culture

The spontaneously dividing CD3⁺, CD4⁺, CD2⁺, CD44⁺ subtype of the ova-specific hybridoma T-cell line called 3DO and mouse thymocytes have been obtained, characterized, and DEX-treated as described before (Ayroldi E et al., 1997; D'Adamio F et al., 1997). For the latter cell type, spleen and lymph node cells were stained with a saturating concentration of FITC-conjugated anti-mouse B220 (clone RA3-6B2; Pharmingen) followed by incubation with α-FITC conjugated magnetic beads (PerSeptive Diagnostic) for 30 minutes. Magnetic separation of the resulting antibody complexes resulted in yields of T cells with purity ≥ 98%. COS-7 cells were maintained in culture in DMEM medium supplemented with 10% FCS.

### Transfection of cultured cells and clone isolation

Transfected clones were prepared as previously described (Nocentini G et al., 1997). Briefly, mouse GILZ cDNA coding sequence (414 base pairs; GenBank acc. n. AF024519) was cloned into a pcDNA3 plasmid (Invitrogen) for expression in 3DO cells. Cells were transfected by electroporation (300 mA, 960 µF) with 15 µg linearized pcDNA3 vector (control clones) or 15 µg linearized pcDNA3 vector expressing the cDNA coding for mouse GILZ (pcDNA3-GILZ). After 36 hours, cells were cultured in medium containing G418 0.8 milligram/millilitre (Gibco), and the cell suspension was plated in 96-wells plates (4 for each transfection). Following 15 to 20 days, no more than 15% of the wells presented alive and growing cells. These cells (pcDNA3-GILZ-3DO) were considered clones after being analysed in ribonuclease protection for the correct expression of exogenous GILZ. The clone GIRL-19 was mostly used but experiments were also repeated in other clones for further validation.

The plasmid expressing Myc-tagged GILZ was a pcDNA3.1/Myc-His vector (Invitrogen), containing the full-length mouse GILZ coding sequence which was PCR amplified and cloned between the *BamHI* and *XbaI* restriction sites.

### TCR-mediated apoptosis assay

Hamster monoclonal α-mouse CD3ε antibody (clone 145-2C11; Pharmingen) was diluted in phosphate-buffered saline (PBS) at 1 microgram/millilitre and distributed in flat-bottomed, high-binding 96 wells plates (Costar), putting 100 microliters for each well). The antibodies were allowed to adhere at 4°C for 20 hours and, after being washed with PBS, the coated wells were used to plate the transfected clones (1 × 10⁵ cells per well) and incubated at 37°C.

### Cell extracts

Nuclear cell extracts were prepared by resuspending 2X10⁷ ice-cold PBS washed cells in 1 millilitre of hypotonic buffer containing HEPES (pH 7.5) 25 milliMolar, KCI 50 milliMolar, Nonidet P-40 (NP-40) 0.5%, dithiothreitol (DTT) 0.1 milliMolar, leupeptin 10 milligrams/millilitre, aprotinin 20 milligrams/millilitre, phenylmethylsulfonyl fluoride (PMSF) 1 milliMolar. The cytoplasmic proteins-containing supernatants, after 10 minutes of incubation on ice, were separated from nuclear pellets by centrifugation. Nuclear pellets were then washed with hypotonic buffer without NP-40 and resuspended in 10 millilitre of lysis buffer (HEPES (pH 7.5) 25 milliMolar, KCI 2 milliMolar, DTT 0.1 milliMolar, leupeptin 10 milligrams/millilitre, aprotinin 20 milligrams/millilitre, PMSF 1 milliMolar). The lysates obtained after 15 minutes of incubation on ice, were diluted with 10 volumes of dilution buffer (HEPES (pH 7.5) 25 milliMolar, glycerol 20%, DTT 0.1 milliMolar, leupeptin 10 milligrams/millilitre, aprotinin 20 milligrams/millilitre, PMSF 1 milliMolar) and cleared in a precooled centrifuge for 30 minutes at 14000 RPM.

Whole cell extracts were prepared by resuspending 2X10⁷ ice-cold PBS washed cells in 1 millilitre of a buffer containing HEPES (pH 7.5) 25 milliMolar, NaCl 150 milliMolar, Igepal CA-630 1 %, MgCl 10 milliMolar, EDTA 1 milliMolar, glycerol 2%, DTT 0.1 milliMolar, leupeptin 10 milligrams/millilitre, aprotinin 20 milligrams/millilitre, PMSF 1 milliMolar, sodium fluoride 25 milliMolar, and sodium orthovanadate 1 milliMolar. The extract, after an incubation of 15 minutes on ice, were cleared in a precooled centrifuge for 30 minutes at 14000 RPM.

### Western blot analysis

The nitrocellulose membranes were prepared and analyzed by Western blot as previously described (Ayroldi E et al., 1997; D'Adamio F et al., 1997). GILZ primary antibodies were a rabbit polyclonal antiserum recognizing GILZ diluted 1:5000, or a monoclonal mouse α-human GILZ antibody prepared by immunizing BaIb/c mices with GST-human GILZ as antigen. The antisera were first screened by ELISA using the antigen and positive spleen cells (cut-off dilution >1:800) were fused with myeloma cells I the presence of feeder cells. Hybridoma supernatants were screened by ELISA and positive cells were cloned by limiting dilution, Some of them were used to inoculate mice intraperitoneally and obtain ascites fluid enriched in monoclonal antibodies. The ascites fluid was heat-inactivated, titered, and stored.

Other primary antibodies, monoclonal mouse α-human c-Fos (Santa Cruz Biotechnology), polyclonal rabbit α-avian c-Jun (Santa Cruz Biotechnology), polyclonal rabbit α-mouse phospho-ERK-1/-2 (Cell Signaling Technology), polyclonal rabbit α-mouse ERK-1/-2 (Cell Signaling Technology), polyclonal rabbit α-human MEK-1 (Cell Signaling Technology), polyclonal rabbit α-human phospho-MEK-1 (Cell Signaling Technology), monoclonal rat α-mouse phospho-Raf (Upstate Biotechnology), polyclonal rabbit α-mouse Raf (Santa Cruz Biotechnology), α-beta tubulin (Calbiochem), were diluted according to manufacturer's instructions. The secondary antibodies were horseradish peroxidase-labeled α-rabbit, α-rat, or α-mouse antibodies (depending on the primary antibody) provided by the SuperSignal chemiluminescence kit (Pierce), used according to manufacturer's instructions. The antibodies against the non-phosphorylated protein variants were used to verify that no modulation of protein expression occurred, whilst α-beta tubulin antibody was used to check that an equivalent amount of proteins were loaded in each lane.

When it was needed to reprobe a membrane with a different primary antibody, the primary and secondary antibodies previously used are "stripped" using the Restore Western Blot Stripping Buffer (Pierce), according to manufacturer's instructions.

### DEX l anti CD3 assay

Murine thymocytes were stimulated for 6 hours with DEX and then for different times with anti-CD3 monoclonal antibodies (from 30 minutes to 3 hours), obtaining similar results. Cell extracts were tested by Western Blot as

### AP-1 luciferase assay

The 3DO cells were transfected with the AP-1 luciferase reporter gene along pcDNA3 (control, empty vector) or pcDNA3-GILZ, with or without the cDNA of the activated form of Raf. Cloned in the pUSEamp vector (Upstate Biotechnology). Each transfection was performed by electroporation as above described in triplicate (5 micrograms of each plasmid). The transfection efficacy was assessed by co-transfeting a plasmid expressing Green Fluorescent Protein. Cell lysis and luciferase quantification were performed using commercial reagents (Roche Diagnostics).

### Cell proliferation assay

The proportion of cells in the different cell cycle phases in the cell proliferation assay was evaluated by propidium iodide solution and flow cytometry. Briefly, cells were centrifuged and the pellets resuspended in 1.5 mL hypotonic propidium iodide (PI) solution. The tubes were kept at 4°C in the dark overnight. The PI-fluorescence of individual nuclei was measured by flow cytometry with standard FACScan equipment (Becton Dickinson). The cell cycle was analysed by Cell Fit program. Transfected clones were prepared as previously described by electroporation (3DO cells) or by using Lipofectamine (H₃₅ rat hepatoma cells) following manufacture's instruction (Gibco BRL), and analyzed after two days. Radiolabeled Thymidine incorporation assay was performed by culturing the cells for 24 hours at serial concentrations (from 1X10⁵ to 0.175X10⁵ per well). 2.5µCi [³H]-thymidine per well were added 15 hours before harvesting with a multiple suction-filtration apparatus (Mash II) on a fiberglass filter (Whittaker Co.) and counted in a β counter (Packard).

### Results

### GILZ overexpression inhibits c-Fos transcription.

It has been demonstrated that recombinant GILZ specifically interacts *in vitro* with c-Fos and c-Jun in vitro, inhibiting the binding of active AP-1 to its target DNA (Mittellstadt PL et Ashwell JD, 2001). It was now addressed the possibility that GILZ could also interfere with the upstream AP-1 activation pathways, such as MAPKs activation and c-Fos and c-Jun transcription.

For this purpose, it was first evaluated, by Western blot, the expression of c-Jun and c-Fos in a 3DO clone over-expressing GILZ upon stimulation with monoclonal α-CD3 antibodies. TCR triggering induced up regulation of c-Fos transcription in the clone transfected with empty vector (Figure 1A, lane 2), but failed to up-regulate c-fos in the clone overexpressing GILZ (Figure 1A, lane 4). No modulation of c-Jun transcription was however observed in both control and GILZ overexpressing clones (Figure 1 B) suggesting the presence of an active constitutive control of the mechanisms responsible for c-Jun transcription in this experimental model. The same results were obtained at all kinetic times tested, ranging from 20 minutes to 6 hours.

### GILZ overexpression inhibits ERK-1/-2 and Raf, but not JNK phosphorylation

The impaired induction of c-Fos protein could explain, in part, the decrease in transactivation of multimerized AP-1, whose transactivation (through c-Fos transcription and c-Jun phosphorylation) is under the control of Ras/MAPKs pathway (Whitehurst CE and Geppert TD, 1996).

Therefore it was tested the possibility that GILZ could interfere the activation of MAPKs ERK-1/2. Cells derived from a control 3DO clone and a GILZ overexpressing 3DO clone were stimulated with monoclonal α-CD3 antibodies for 5 and 60 minutes. As expected, the control clone displayed increasing level of ERK-1/2 phosphorylation upon activation. In contrast, the GILZ overexpressing clone failed to respond to α-CD3 triggering (Figure 2A). Phosphorylation of Raf showed the same behaviour (Figure 2B). These results were reproduced using different 3DO clones overexpressing GILZ.

The effect of GILZ is inducible by DEX and correlates with the inhibition of anti-CD3 induced signalling in murine thymocytes, in particular, with the phosphorylation of Raf and of downstream proteins MEK and ERK-1/-2, which was reduced in a coordinated manner (figure 3). These results were reproduced, also in COS-7 cells overexpressing a myc-GILZ fusion protein and stimulated with phorbol 12-myristate 13-acetate (PMA), where the inhibition of MEK phosphorylation was demonstrated using Western blot analysis and anti-pMEK-1/-2 antibodies.

JNK, which controls C-Jun phosphorylation and transcription and whose activation is under control of stress-activated MAPKs pathway, was already phosphorylated in non-stimulated 3DO clones (both empty- and GILZ transfected-clones). The stimulation with α-CD3 monoclonal antibodies did not augment JNK phosphorylation that, on the contrary, decreased by the time (Figure 4). The lack of modulation of JNK activation well matched with the lacked modulation of c-Jun transcription observed in the experiment showed in figure 1.

### Effects of GILZ or Raf overexpression on the transcription activity mediated by AP-1

The effects of GILZ on AP-1 transcriptional activity were tested in absence or in presence of activated Raf using transient transfection of 3DO cells with plasmid expressing luciferase under the control of an AP-1 regulated promoter. This vector was transfect with an empty vector or a vector expressing GILZ, showing the inhibiting activity of GILZ on AP-1 mediated transcription under anti-CD3 activation (figure 5A). However, if the activation is performed in cells co-transfected with a plasmid expressing an activated form of Raf, the AP-1 mediated transcription is re-established at the original levels (Figure 5B). The same cell extracts, if tested by Western blot as before (see figure 3), show also the re-establishment of normal phosphorylation levels of ERK-1/-2 and MEK. Therefore, an overexpression of Raf overcomes GILZ inhibitory effects, showing the specificity of GILZ properties.

These data indicate that GILZ inhibits Raf, MEK, and ERK-1/-2 phosphorylation and the consequent c-Fos/AP-1 mediated transcription, strongly suggesting that the failure to synthesise Fos proteins is responsible for the impaired formation of AP-1 heterodimeric complexes and thus for the impaired AP-1 transactivation.

### GILZ overexpression and cell proliferation

The plasmid allowing the overexpression of GILZ (pcDNA3-GILZ) was transfected in various cell types to verify if GILZ, in view of the previous experiments showing an effect on the signal transduction pathway controlling cell proliferation, has an effect on this cell function. The data observed in some cell lines (stable or transiently transfected) confirmed the anti proliferative activity of GILZ, which drives the accumulation of cells in phase G₀G₁ of cell cycle (Table III). These evidences on the control of cell proliferation mediated by GILZ were also verified also by measuring the uptake of radiolabeled thymidine.

### Example 2: mechanisms of the GILZ-mediated inhibition of the Raf-controlled MAPKs transduction pathway.

### Methods

### Immunoprecipitations.

Immunoprecipitations were performed in RIPA buffer (TRIS (pH 7.5) 50 milliMolar, NaCl 150 milliMolar, Nonidet P-40 1%, deoxycholate 0.5%, sodium dodecyl sulphate (SDS) 0.1%, and EDTA 5 milliMolar) supplemented with 1 mM PMSF.

For the immunoprecipitation using whole cell extracts of mouse cells (spleen, lymph nodes, thymocytes and 3DO cells), α-Raf, α-NF-AT, and α-Ras antibodies (Upstate Technology) were used at the concentration of 8 micrograms for each milligram of protein extracts. Antigen-antibody complexes were precipitated with protein A-Sepharose beads (Pharmacia) and dissociated from beads prior to SDS-PAGE by boiling in loading buffer.

For the immunoprecipitation using whole cell extracts of COS-7 cells, the cells were transfected by the DEAE-dextran method as previously described (Luo ZJ et al., 1995), using 2 micrograms of each plasmid. The plasmid expressing human Raf was a pUSEamp vector (Upstate Biotechnology). COS-7 lysates (500 µg) were immunoprecipitated in RIPA buffer with α-Myc antibodies (4 µg/mg protein, Invitrogen) and western blot performed with α-Myc antibodies (1 µg/ml, Invitrogen,) or α-Raf antibodies (Upstate Biotechnology). The Western blots were performed as described above.

### GST fusion proteins

Glutathione S-transferase GILZ fusion protein (GST-GILZ) was prepared as previously described (Ayroldi E et al., 2001). GST-Raf-RBD, the GST fusion protein comprising the Ras Binding Domain of human Raf (Raf-RBD, residues 1-149; De Rooij J and Bos JL, 1997) was cloned in the same expression vector pGEX-4T2 plasmid (Pharmacia) and obtained in the same way.

### GST pull-down experiments.

Extracts were prepared from the indicated cell, treated or untreated with DEX (10 microMolar), as previously described (Ayroldi E et al., 2001). GST or GST fusion proteins, loaded on Sepharose beads, were mixed with cell extracts in binding buffer (NaCl 250 miliiMolar, HEPES (ph7.5) (pH 7.5) 50 milliMolar, EDTA 0.5 milliMolar, Nonidet P-40 0.1% (v/v), phenylmethylsulfonyl fluoride (PMSF) 0.2 milliMolar, dithiothreitol (DTT) 1 milliMolar, bovine serum albumin (BSA) 100 µg/ml), heated for 5 minutes at 42 °C, incubated for 2 hours at 4°C, washed extensively with binding buffer, resuspended in loading buffer and analysed by SDS-PAGE and Western blot as described above. In the case of murine thymocytes extracts, the incubation with the beads was performed overnight and at 4°C. The α-MEK and α-ERK antibodies (Upstate Biotechnology) were used following manufacturer's instructions.

*In vitro* translated proteins were diluted with binding buffer (HEPES (pH 7.5) 25 milliMolar, glycerol 10%, NaCl 50 milliMolar, Nonidet P-40 0.05%, 1 mM DTT) and pre-cleared with glutathione beads for 45 minutes at 4°C. GST or GST fusion proteins were bound to glutathione beads and incubated with *in vitro* translated proteins for 18 hours at 4°C. The beads were subsequently washed five times with 0.5 millilitre of PBS and the proteins recovered by boiling the beads in SDS sample buffer were analysed by SDS-PAGE.

### Results

### GILZ interacts with Raf and Ras.

Antigen-induced activation leads to conversion of Ras to its active form as well as activation of the kinase Raf. It has also been shown that treatment of mast cells with DEX blocked the phosphorylation of Raf, MEK, ERK-2 without affecting Ras activation (Cissel DS and Beaven MA, 2000). Since protein-protein interaction may have important consequences on protein phosphorylation, activation and trafficking, it has to be demonstrated if a DEX-induced protein such as GILZ is eventually capable of binding proteins belonging to the MAPKs cascade and to inhibit their activation.

Initially, GST-GILZ, containing entire mouse GILZ, was immobilised on beads and used in pull-down experiments with protein extracts obtained from DEX un-/stimulated 3DO cells, using beads loaded with GST as a control. The proteins interacting with the beads were separated on a SDS-PAGE gel and transferred on a membrane then probed with an α-GILZ antibody. A band immunoreactive with α-Raf antibodies is clearly detectable only when GST-GILZ is used in the pull-down experiment (figure 6A). These results were reproduced with with α-Ras antibodies (figure 6B), as well as in protein extracts obtained from thymocytes and from purified T cells isolated in spleen and lymph nodes, without significant differences in binding between both untreated and DEX treated cells.

The initial hypothesis was further tested by using murine thymocytes, known to upregulate GILZ expression upon DEX stimulation. Murine thymocytes were treated for 6 hours with DEX and the whole cell lysates were immunoprecipitated with antibodies recognizing either Raf or NF-AT. The immunoprecipitated material was analyzed by Western blot using antibodies recognizing either GILZ or Raf. The antibody against GILZ detects an immunoreactive protein only in the lysates immunoprecipitated with the α-Raf antibody and not with the α-NF-AT antibody. Moreover, a higher amount of GILZ is co-immunoprecipitated from DEX-stimulated cells, as expected from the increased expression of GILZ in such cells (Figure 7A). As shown in the Western blot generated with the α-Raf antibody, Raf expression in murine thymocytes seems not affected by the treatment with DEX (Figure 7B), therefore the increased amount of immunoprecipitated GILZ results directly by DEX induction. Similar results were obtained using antibodies against Ras in immunoprecipitation and Western blot.

The GILZ interaction with Ras / Raf was also demonstrated by means of a different cell assay. A cell line (COS-7) was transfected with a plasmid expressing Raf with or without another plasmid expressing GILZ fused with Myc, an epitope helping the independent detection of GILZ. The extracts obtained from the transfected cells were used in immunoprecipitation experiments where antibodies specific for Myc were applied. Wherein Raf is detectable in all the whole lysates, the α-Myc antibody immunoprecipitates Raf in the whole cell lysates transfected with the plasmid expressing myc-GILZ only when the plasmid expressing Myc-GILZ is co-transfected. As expected, the α-Myc antibody detects myc-GILZ protein in the whole lysate of cells transfected with myc-GILZ and Raf, as well as in material co-immunoprecipitated with α-Raf antibodies, but not in cell extracts obtained from cells transfected only with the plasmid expressing Raf (figure 8). As before, similar results were obtained using antibodies against Ras in immunoprecipitation and Western blot.

For a comparative test, the N-terminus of Raf (residues 1-149) has been also expressed as a fusion protein with GST. This Raf segment contains the Ras binding domain of Raf (RBD, residues 51-131, Winkler DG et al., 1998) and was then called GST-Raf-RBD.

In a first assay, cell extracts from a COS-7 cell line overexpressing Ras were incubated with GST-Raf-RBD and an increasing amount of *E*. *coli* expressed mouse GILZ. After washing, the complexes were analysed by Western blot, demonstrating dose-dependent displacement effect of Ras from GST-Raf-RBD due to GILZ (figure 9A). A second GST pull-down experiments also show that, whereas no GILZ is detectable with GST-protein alone, a protein recognized by antibodies against GILZ can be detected in the material retained by immobilised GST-Raf-RBD in the whole cell lysates (figure 9B). As shown in the previous immunoprecipitation experiments with antibodies against Raf (figure 7A), a larger amount of GILZ is detected in the extracts from the DEX-treated thymocytes.

These experiments demonstrate that GILZ interact with the Raf domain also involved with the recognition of Ras, possibly by competing with Ras. However this interfering effect may be also exerted in the trimeric complex GILZ / Raf /Ras

Finally, the specificity of the binding of GILZ for Raf instead for other components of the same pathway was also tested using the pull-down assay described before with lysates obtained from un-/stimulated murine thymocytes, and α-MEK and α-ERK-1/-2 antibodies for the Western Blot. In this case, the antibodies did not reveal a significant presence of these kinases in the material pulled down with GILZ, excluding a direct interaction of GILZ with these proteins (figure 10).

These evidences corroborates the hypothesis that protein-protein interaction involving GILZ are responsible for changes in the transcriptional pattern not only at the level of protein directly involved in DNA binding, but also at the level of upstream regulators of transcription factors, in particular by suppressing the phosphorylation of Raf and, consequently, of the down stream phosphorylation of MAPKs..

### Example 3: Structure-function study of the GILZ/Raf interaction.

### Methods

### GST fusion proteins including GILZ fragments and GST pull-down experiments

Glutathione S-transferase fusion protein including diffrent segments of GILZ were prepared by cloning the segment encoding for such GILZ fragments in the plasmid originally described for the expression of GST-GILZ (Ayroldi E et al., 2001). When necessary (i.e. whenever the original GILZ methionine was not included), a Met codon was added by at 5' of the sequence by normal genetic. The GST pull-down experiments were performed as described in the previous example with 3DO cells.

### Radiolabeled GILZ proteins

Full, deleted, and mutated mouse GILZ (figure 11) were obtained by PCR and cloned in pCR3.1 (Invitrogen). ΔC-GILZ contains the first 97 amino acids of mouse GILZ. DN-GILZ contains the first 8 amino acids of mGILZ fused with the residues 73-137. The *in vitro* translation with [³⁵S]-Methionine was performed with a commercial rabbit reticulocyte transcription-translation system (TNT, Promega).

### Results

### Specificity of the GILZ/Raf interaction.

In view of the results on the inhibiting effect of GILZ overexpression on ERK-1/-2 phosphorylation and of physical interaction between GILZ and Raf, it is important to identify the GILZ and Raf protein domains responsible for the binding. Amongst the several approaches commonly used to study protein-protein interactions, pull-down experiments making use of GILZ and Raf proteins fused to with Glutathione-S-Transferase (GST) were performed.

An *in vitro* structure-activity study has been performed by assaying cell lysates in pull-down experiments, as described before, with a series of truncated or single site mutants of GILZ and Raf, as well as *in vitro* 35^{S}-labeled GILZ mutants, in order to map the domains of these proteins involved in the interaction more precisely (figure 11).

GST-pull-down experiments were also performed using the recombinant GST-Raf-RBD fusion protein (Figure 12). GST-Raf-RBD fusion protein, but not GST alone, binds GILZ full-length protein as well as the GILZ truncated form missing the C-terminal region (ΔC-GILZ). On the contrary, beads loaded with GST-Raf-RBD fusion protein do not retain the GILZ form missing the N-terminal region (ΔN-GILZ).

These two truncated forms of GILZ were designed to both comprise residues 76-97, which includes a Leucine zipper known to be involved in GILZ dimerization. Specific mutations were then introduced in this segment to check if GILZ properties of homodimerization and Raf interaction are provided by the same protein sequence or by distinct domains. Recombinant full GILZ proteins wherein either an Asparagine (N87A-GILZ) or three leucine (3LA-GILZ) are mutated in Alanine are not retained by GST-GILZ immobilized on beads, confirming the importance of these residues in GILZ homodimerization. However, these two mutants are retained by the beads where GST-Raf-RBD is immobilized (figure 13). These experiments suggest that dimerization and Raf binding are GILZ properties due to different protein domains.

Finally, GST fusion proteins including various segments of the N-terminal domain of GILZ were tested in 3DO as previously described (see figure 6A). It appears that GILZ fragments containing at least the region comprised between residues 16 and 36 (SEQ ID NO: 3) bind efficiently Raf (figure 14).

### Structure-activity predictions based on GILR sequence

As previously discussed, TSC protein family comprises leucine zipper proteins (such as GILZ, TSC-22, THG-1, DIP) sharing an evolutionary conserved dimerization domain comprised between less conserved N-terminal and C-terminal domains. These latter ones are probably the regions characterising the functions of TSC proteins, meanwhile the conserved one allow the homodimerization and, possibly, heterodimerization of these proteins.

The only TSC-related protein whose structure has been solved is porcine DIP (Seidel G et al., 1997), a 77-residue long protein which is highly homologous to the segment 58-134 of human GILZ, corresponding to central / C-terminal portion of GILZ and TSC-22. Therefore, no structure data are available for most of the area delimited by the previous experiments as the Raf binding domain of GILZ.

Several methods for predicting the secondary structure of proteins are available and some of them have been applied to mGILZ(1-97) and hGILZ (1-97). On the basis of the predictions, this N-terminal region comprises an helical region internal to a random coiled area (residues 1-20), an area in which extended strands are strongly predicted (residues 21-50), and a long helical structure including the four key leucine residues (at positions 76, 83, 90 and 97) and an asparagine residue (at position 87) within the leucine zipper domain, which are compatible with the canonical leucine zipper structure of the family (figure 15). According to PROSITE database (Bairoch A et al., Nucl. Acids Res 1997, 25: 217-221; URL: http://www.expasy.ch/prosite), this latter segment contains the beginning of the TSC protein family signature (at Methionine 58) and a leucine-zipper pattern (starting on Leucine 76).

The GILZ sequence allowing the Raf binding is contained in GILZ(16-36), as shown in figure 14. In particular, GILZ binding determinants structural features may be present in the most N-terminal and C-terminal sequences of this fragment, since the central region is highly hydrophobic (see the sequence STSFFSSLL between 21 and 29). The examples provided in the prior art for other protein complex regulating apotosis or transformation, such asMDM2/p53 and BcIXL/Bak, show that helix coiled coil peptides can be used as isolated molecules and modified to develop compounds disrupting protein-protein interactions (Cochran AG 2001; Chin JW and Schepartz A, 2001).

It can be inferred that polypeptides or peptides comprising at least 5 consecutive amino acids of SEQ ID NO: 3 are fragments of the N-terminal domain of GILZ corresponding to structural elements of such region. Examples of these peptides correspond to the sequences GILZ(1-20), GILZ(21-50), GILZ (1-50), GILZ (10-30), GILZ (10-40), GILZ (16-22), GILZ (30-36), GILZ (10-50), GILZ (30-50), GILZ(16-58), or GILZ(1-36).

Therefore GILZ fragments including, partly or completely, sequences belonging to one or more of these structural elements can be usefully tested. Such fragments should display the same novel biological activity of GILZ characterized in the present invention, as determined by means of routine experimentation comprising subjecting such an analog to the assays disclosed in the present application.

The results obtained with GILZ mutants indicate that the N-terminal domain of GILZ interacts with the Ras binding domain of Raf. Moreover, the results obtained using non-dimerising GILZ mutants also indicate that, different from GILZ/NF-kB interaction, GILZ/Raf interaction does not require GILZ dimerisation and that it is compatible with a 1:1, protein to protein, interaction model. Compared with the evidences on the GILZ interaction with NF-kB, it can be suggested that different molecular portions are responsible for the interaction with NF-kB and Raf and that, possibly, GILZ could preferentially bind to NF-kB or Raf depending on the ratio of its dimeric and/or monomeric arrangement. The obtained results cannot also exclude that GILZ may bind Raf alone or together with other Raf interacting proteins (such Ras, as demonstrated in example 2). depending on the cell state (un-/stimulated, un/transformed) and/or on the cell type (peripheral/ central lymphocyte, or other tissues)

The above results clearly demonstrate that GILZ is directly involved in the GCH-induced MAPK pathway inhibition as a main mediating agent of the MAPKs-mediated effects, modulating the cell proliferation / inflammatory / immunosuppressive activity of the cells, and possibly cytokine production.

**TABLE I**

| **Amino Acid** | **Synonymous Group** | **More Preferred Synonymous Groups** |
|---|---|---|
| **Ser** | Gly, Ala, Ser, Thr, Pro | Thr, Ser |
| **Arg** | Asn, Lys, Gln, Arg, His | Arg, Lys, His |
| **Leu** | Phe, Ile, Val, Leu, Met | Ile, Val, Leu, Met |
| **Pro** | Gly, Ala, Ser, Thr, Pro | Pro |
| **Thr** | Gly, Ala, Ser, Thr, Pro | Thr, Ser |
| **Ala** | Gly, Thr, Pro, Ala, Ser | Gly, Ala |
| **Val** | Met, Phe, Ile, Leu, Val | Met, Ile, Val, Leu |
| **Gly** | Ala, Thr, Pro, Ser, Gly | Gly, Ala |
| **Ile** | Phe, Ile, Val, Leu, Met | Ile, Val, Leu, Met |
| **Phe** | Trp, Phe,Tyr | Tyr, Phe |
| **Tyr** | Trp, Phe,Tyr | Phe, Tyr |
| **Cys** | Ser, Thr, Cys | Cys |
| **His** | Asn, Lys, Gln, Arg, His | Arg, Lys, His |
| **Gln** | Glu, Asn, Asp, Gln | Asn, Gln |
| **Asn** | Glu, Asn, Asp, Gln | Asn, Gln |
| **Lys** | Asn, Lys, Gin, Arg, His | Arg, Lys, His |
| **Asp** | Glu, Asn, Asp, Gln | Asp, Glu |
| **Glu** | Glu, Asn, Asp, Gln | Asp, Glu |
| **Met** | Phe, Ile, Val, Leu, Met | Ile, Val, Leu, Met |
| **Trp** | Trp, Phe, Tyr | Trp |

**TABLE II**

| **Amino Acid** | **Synonymous Group** |
|---|---|
| **Ser** | D-Ser, Thr, D-Thr, allo-Thr, Met, D-Met, Met(O), D-Met(O), L-Cys, D-Cys |
| **Arg** | D-Arg, Lys, D-Lys, homo-Arg, D-homo-Arg, Met, Ile, D-.Met, D-Ile, Orn, D-Orn |
| **Leu** | D-Leu, Val, D-Val, AdaA, AdaG, Leu, D-Leu, Met, D-Met |
| **Pro** | D-Pro, L-I-thioazolidine-4-carboxylic acid, D-or L-1-oxazolidine-4-carboxylic acid |
| **Thr** | D-Thr, Ser, D-Ser, allo-Thr, Met,D-Met, Met(O), D-Met(O), Val, D-Val |
| **Ala** | D-Ala, Gly, Aib, B-Ala, Acp, L-Cys, D-Cys |
| **Val** | D-Val, Leu, D-Leu, Ile, D-Ile, Met, D-Met, AdaA, AdaG |
| **Gly** | Ala, D-Ala, Pro, D-Pro, Aib, .beta.-Ala, Acp |
| **Ile** | D-Ile, Val, D-Val, AdaA, AdaG, Leu, D-Leu, Met, D-Met |
| **Phe** | D-Phe, Tyr, D-Thr, L-Dopa, His, D-His, Trp, D-Trp, Trans-3,4, or 5-phenylproline, AdaA, AdaG, cis-3,4, or 5-phenylproline, Bpa, D-Bpa |
| **Tyr** | D-Tyr, Phe, D-Phe, L-Dopa, His, D-His |
| **Cys** | D-Cys, S--Me--Cys, Met, D-Met, Thr, D-Thr |
| **Gln** | D-Gln, Asn, D-Asn, Glu, D-Glu, Asp, D-Asp |
| **Asn** | D-Asn, Asp, D-Asp, Glu, D-Glu, Gln, D-Gln |
| **Lys** | D-Lys, Arg, D-Arg, homo-Arg, D-homo-Arg, Met, D-Met, Ile, D-Ile, Orn, D-Orn |
| **Asp** | D-Asp, D-Asn, Asn, Glu, D-Glu, Gln, D-Gln |
| **Glu** | D-Glu, D-Asp, Asp, Asn, D-Asn, Gln, D-Gln |
| **Met** | D-Met, S--Me--Cys, Ile, D-Ile, Leu, D-Leu, Val, D-Val |

**TABLE III**

| **CELLS** | **GROUPS** | **G₀**G**₁** | **S** | **G₂**M |
|---|---|---|---|---|
| **COS-7** | pcDNA3 (15 micrograms) | 19.3% | 64.8 | 15.9% |
| | pcDNA3-GILZ (5 micrograms) | 18.5% | 64% | 17.5% |
| | pcDNA3-GILZ (15 micrograms) | 54% | 13.5% | 32.5% |
| **H₃₅ cell line (rat hepatoma)** | pcDNA3(2.5 micrograms) | 31.4% | 51.4% | 17.2% |
| | pcDNA3 (2.5 micrograms) | 40.2% | 37.2% | 22.6% |
| **3DO (Stable clones)** | Control | 25% | 66%% | 9% |
| | Clone GIRL-19 | 35% | 56.3% | 6.7% |

### REFERENCES

Ashwell JD et al., Annu Rev Immunol, 2000, 18: 309-345.
Ayroldi E et al., Blood, 1997, 89: 3717-3726.
Ayroldi E et al., Blood, 2001, 98: 743-753.
Barnard D et al., Biochem Biophys Res Commun 1998, 247:176-80.
Bell AJ Jr et al., Protein Eng 2002, 15: 817-25.
Block C et al., Nat Struct Biol, 1996, 3: 244-51.
Bodor et al., Science 1992, 257: 1698-1700.
Brown A et al., J Pept Sci, 1996, 2: 40-6.
Cannarile L et al., Cell Death Differ 2001, 8: 201-3.
Chin JW and Schepartz A, Angew Chem Int (Ed Engl), 2001, 40: 3806-3809.
Cissel DS and Beaven MA, J Biol Chem, 2000, 275: 7066-70.
Cleland JL et al., Curr Opin Biotechnol 2001,12: 212-9.
Cochran AG, Curr Opin Chem Biol 2001, 5: 654-659.
D'Adamio F et al., Immunity 1997, 7: 803-812.
De Bosscher K et al., Proc Natl Acad Sci USA 1997, 94: 13504-09.
De Rooij J and Bos JL, Oncogene, 1997, 14: 623-5.
Dougherty DA, Curr Opin Chem Biol 2000, 4: 645-52.
Edgerton MD et al., Methods Mol Biol, 2000, 138: 33-40.
Feng A et al., J Cell Biol 1995, 131: 1095-1103.
Filikov AV et al., Protein Sci 2002, 11: 1452-61.
Fiorenza MT et al., Gene 2001, 278: 125-30.
Friden et al., Science 1993, 259: 373-377.
Gabb HA et al., J Mol Biol 1997, 272:106-20.
Golebiowski A et al., Curr Opin Drug Discov Devel 2001, 4: 428-34.
Grabenhorst E et al., Glycoconj J 1999, 16: 81-97.
Hayes RJ et al., Proc Natl Acad Sci U S A, 2002, 99: 15926-31.
Hino S et al., Biochem Biophys Res Commun, 2000, 278: 659-64.
Hino S et al., Biochem Biophys Res Commun 2002 , 292: 957-63.
Hruby VJ and Balse PM, Curr Med Chem 2000, 7: 945-70.
Imperiali B and O'Connor SE, Curr Opin Chem Biol 1999, 3: 643-9.
Jamieson C and Yamamoto KR, Proc Natl Acad Sci USA 2000, 97: 7319-7324.
Jay P et al., Biochem Biophys Res Commun 1996, 222: 821-826.
Jehn BM and Osborne BA, Crit Rev Eukaryot Gene Expr 1997, 7:179-193.
Kane JF, Curr Opin Biotechnol 1995, 6: 494-500.
Kelley LA et al., J Mol Biol 2000, 299: 499-520.
Kester HA et al., J Biol Chem 1999, 274: 27439-27447.
Khanna C et al., Cancer Res 2001, 61: 3750-3759.
Kim HO and Kahn M, Comb Chem High Throughput Screen 2000; 3: 167-8.
Kloog Y and Cox AD, Mol Med Today 2000, 6: 398-402.
Kolch W, Biochem J 2000, 351: 289-305.
Kraemer-Pecore CM et al., Cur Opin Chem Biol, 2001, 5: 690-695.
Luo B and Prestwich GD, Exp Opin Ther Patents 2001, 11: 1395-1410.
Luo ZJ et al., J Biol Chem, 1995, 40: 23681-87.
Maruta H et al., Methods Mol Biol, 2002, 189: 75-85.
McCormick SM et al., Proc Natl Acad Sci USA 2001, 98: 8955-8960.
Mittelstadt PR and Ashwell JD, J Biol Chem 2001, 276: 29603-10.
Murphy LR et al., Protein Eng. 2000, 13:149-52.
Nocentini G et al., Proc Natl Acad Sci USA, 1997, 94: 6216-6221
Ohnishi M et al., J Biol Chem, 1998, 273: 10210-5.
Pillai O and Panchagnula R, Cur Opin Chem Biol, 5: 447-451, 2001
Radziwill G et al., Biochem Biophys Res Commun, 1996, 227: 20-6.
Ramdas J and Harmon JM, Endocrinology 1998, 139: 3813-21.
Ranney DF, Biochem Pharmacol 2000, 59: 105-14.
Riccardi C et al., Therapie 2000, 55: 165-9.
Rider LG et al., J Immunol 1996, 157: 2374-80.
Rincon M, Current opinion Immunol 2001, 13: 339-345.
Rogov SI and Nekrasov AN, Protein Eng 2001, 14: 459-463.
Sebolt-Leopold JS; Oncogene 2000, 19: 6594-9.
Seidel G et al., J Biol Chem 1997, 272: 30918-30927.
Shibanuma M et al., J. Biol. Chem 1992, 267, 10219-10224).
Sillard R et al., Eur J Biochem 1993, 216: 429-436.
Spiro RG, Glycobiology 2002, 12: 43R-56R.
Thanka Christlet TH and Veluraja K, Biophys J 2001, 80: 952-60.
Vacchio MS et al., J Exp Med 1994, 179: 1835-46.
Villain M et al., Chem Biol, 8(7):673-9, 2001.
Vita C et al., J Immunol Methods, 266: 53-65, 2002.
Vogel P et al., Biochim Biophys Acta 1996, 1309: 200-204.
Weinstein-Oppenheimer CR et al., Pharmac Therap 2000, 88: 229-279.
Whitehurst CE and Geppert TD, J Immunol, 1996, 156: 1020-9.
Widen C et al., J Biol Chem 2000, 275: 39296-01.
Williams JG et al., J Biol Chem 2000, 275: 22172-9.
Winkler DG et al., J Biol Chem, 1998, 273: 21578-84.
Wu RZ et al., Trends Cell Biol 2002, 12: 485-8.
Zeng J et al., Protein Eng 2001, 14: 39-45.

## Claims

1. A peptide capable of binding to Raf protein and of inhibiting the MAPKs pathway selected from:
(a) a peptide consisting of the amino acid sequence 1 to 50 of SEQ ID NO: 1 or SEQ ID NO:2;
(b) fragment of the peptide according to (a) containing at least 10 amino acids, preferably at least 20 amino acids;
(c) fragment according to (b) having a sequence corresponding to amino acid residues 1 to 20, 21 to 50, 10 to 30 or 10 to 50 of SEQ ID NO: 1 or SEQ ID NO: 2.

2. A peptide capable of binding to Raf protein and of inhibiting the MAPKs pathway selected from:
(a) a peptide consisting of the amino acid sequence SEQ ID NO: 3;
(b) fragment of the peptide according to (a) comprising at least 5 consecutive amino acids of SEQ ID No: 3;
(c) fragment according to (b) consisting of a sequence corresponding to amino acid residues 1 to 7 or 15 to 21 of SEQ ID NO: 3.

3. A peptide according to claim 1 or 2 in the forms of active fractions, precursors, salts, or derivatives. Derivatives can be prepared from the functional groups present on the lateral chains of the amino acid moieties or on the terminal N- or C- groups.

4. Peptide mimetics of the peptides according to any one of claims 1 to 3.

5. The peptide according to any one of claims 1 to 4 chemically modified with molecules that, being transported naturally across the cell membrane, facilitate their entry or enhance their permeability across the cell membrane and into the cytoplasm.

6. DNA sequence encoding the GILZ-derived peptides according to claim 1 or 2.

7. An expression vector comprising the DNA of claim 6, together with any appropriate stop / start transcription and translation elements and any other additional sequence.

8. A peptide according to any one of claims 1 to 7 for use as a medicament.

9. A pharmaceutical composition comprising the compound according to any one of claims 1 to 8, as active ingredient and a pharmaceutically acceptable carrier, excipient, stabilizer or diluent.

10. A host cell transformed with the expression vector of claim 7.

11. A purified preparation of a peptide according to any one of claims 1 to 5 which contains at least 1% of said compound.

12. A method for screening *in vitro* compounds possibly binding to Raf and inhibiting MAPKs pathway, by comparing the effect of such compounds with the effect provided by the peptides according to any one of claims 1 to 3.

13. A kit for screening *in vitro* and *in vivo* compounds possibly binding to Raf and inhibiting MAPKs pathway, by comparing the effect of such compounds with the effect provided by the peptides according to any one of claims 1 to 3.

14. A method for inhibiting unwanted cell proliferation mediated by the MAPKs in cultured cells by administering an effective amount of a peptide according to any one of claims from 1 to 5.

15. Use of a peptide according to any one of claims 1 to 7 for the manufacture of a medicament for the treatment of cancer.

16. Use according to claim 15 in which the cancer is selected form the group consisting of carcinomas, hematopoietic tumors of lymphoid/myeloid lineage, lymphomas, lymphocytic leukemia, tumors of mesenchymal origin and melanoma.

17. Use of a peptide according to any one of claims 1 to 7 for the manufacture of a medicament for the treatment of autoimmune or inflammatory diseases.

18. Use according to claim 17 in which the autoimmune or inflammatory disease is selected form the group consisting of systemic lupus erythematosus (SLE), type 1 diabetes, vasculitis, autoimmune chronic active hepatitis, ulcerative cholitiss, Crohn's disease, allergic diseases, nephritic syndrome, sarcoidosis, and rheumatoid arthritis.

19. Use of a peptide according to any one of claims 1 to 7 for the manufacture of a medicament for allograft or xenograft recipients to prevent rejection of a transplanted organ.

20. A peptide consisting of the amino acid sequence SEQ ID NO: 1 or SEQ ID NO:2 for use as a medicament.

21. A method for screening *in vitro* and *in vivo* compounds possibly binding to Raf and inhibiting MAPKs pathway, by comparing the effect of such compounds with the effect provided by the peptides consisting of the amino acid sequence SEQ ID NO: 1 or SEQ ID NO:2.

22. A kit for screening *in vitro* and *in vivo* compounds possibly binding to Raf and inhibiting MAPKs pathway, by comparing the effect of such compounds with the effect provided by the peptides consisting of the amino acid sequence SEQ ID NO: 1 or SEQ ID NO:2.

23. Use of a peptide consisting of the amino acid sequence SEQ ID NO: 1 or SEQ ID NO:2 for the manufacture of a medicament for the treatment of cancer.

24. Use according to claim 23 in which the cancer is selected form the group consisting of carcinomas, hematopoietic tumors of lymphoid/myeloid lineage, lymphomas, lymphocytic leukemia, tumors of mesenchymal origin and melanoma.

25. Use of a peptide consisting of the amino acid sequence SEQ ID NO: 1 or SEQ ID NO:2 for the manufacture of a medicament for the treatment of autoimmune or inflammatory diseases.

26. Use according to claim 25 in which the autoimmune or inflammatory disease is selected form the group consisting of systemic lupus erythematosus (SLE), type 1 diabetes, vasculitis, autoimmune chronic active hepatitis, ulcerative cholitiss, Crohn's disease, allergic diseases, nephritic syndrome, sarcoidosis, and rheumatoid arthritis.

27. Use of a peptide consisting of the amino acid sequence SEQ ID NO: 1 or SEQ ID NO:2 for the manufacture of a medicament for allograft or xenograft recipients to prevent rejection of a transplanted organ.

## Patentansprüche

1. Peptid, das fähig ist, an das Raf-Protein zu binden und den MAPKs-Weg zu inhibieren, ausgewählt aus:
(a) einem Peptid, das aus der Aminosäuresequenz 1 bis 50 der SEQ ID NO:1 oder SEQ ID NO:2 besteht;
(b) Fragment des Peptids nach (a), das mindestens 10 Aminosäuren, bevorzugt mindestens 20 Aminosäuren enthält;
(c) Fragment nach (b), das eine Sequenz entsprechend den Aminosäurresten 1 bis 20, 21 bis 50, 10 bis 30 oder 10 bis 50 der SEQ ID NO:1 oder SEQ ID NO:2 hat.

2. Peptid, das fähig ist, an das Raf-Protein zu binden und den MAPKs-Weg zu inhibieren, ausgewählt aus:
(a) einem Peptid, das aus der Aminosäuresequenz SEQ ID NO:3 besteht;
(b) Fragment des Peptids nach (a), das mindestens 5 aufeinanderfolgende Aminosäuren der SEQ ID NO:3 umfasst;
(c) Fragment nach (b), das aus einer Sequenz entsprechend den Aminosäurresten 1 bis 7 oder 15 bis 21 der SEQ ID NO:3 besteht.

3. Peptid nach Anspruch 1 oder 2 in den Formen von aktiven Fraktionen, Vorläufern, Salzen oder Derivaten. Derivate können aus den funktionellen Gruppen, die auf den Seitenketten der Aminosäurereste oder auf den terminalen N- oder C-Gruppen vorhanden sind, hergestellt werden.

4. Peptidmimetika der Peptide nach einem der Ansprüche 1 bis 3.

5. Peptid nach einem der Ansprüche 1 bis 4, das mit Molekülen, die natürlicherweise durch die Zellmembran transportiert werden, die ihren Eintritt erleichtern oder die ihre Durchlässigkeit durch die Zellmembran und in das Cytoplasma verbessern, chemisch modifiziert ist.

6. DNA-Sequenz, die die von GILZ abgeleiteten Peptide nach Anspruch 1 oder 2 kodiert.

7. Expressionsvektor umfassend die DNA nach Anspruch 6 zusammen mit irgendwelchen geeigneten Stop/Start-Transkriptions- und Translationselementen und irgendeiner anderen zusätzlichen Sequenz.

8. Peptid nach einem der Ansprüche 1 bis 7 zur Verwendung als Medikament.

9. Pharmazeutische Zusammensetzung umfassend die Verbindung nach einem der Ansprüche 1 bis 8 als aktiven Inhaltsstoff und einen pharmazeutisch akzeptablen Träger, Hilfsstoff, Stabilisierungsmittel oder Verdünnungsmittel.

10. Wirtszelle, die mit dem Expressionsvektor nach Anspruch 7 transformiert ist.

11. Aufgereinigte Zubereitung eines Peptids nach einem der Ansprüche 1 bis 5, die mindestens 1 % der Verbindung enthält.

12. Verfahren zum *in vitro* Screening von Verbindungen, die möglicherweise an Raf binden und den MAPKs-Weg inhibieren, indem die Wirkung derartiger Verbindungen mit der Wirkung, die durch die Peptide nach einem der Ansprüche 1 bis 3 bereitgestellt wird, verglichen wird.

13. Kit zum *in vitro* und *in vivo* Screening von Verbindungen, die möglicherweise an Raf binden und den MAPKs-Weg inhibieren, indem die Wirkung derartiger Verbindungen mit der Wirkung, die durch die Peptide nach einem der Ansprüche 1 bis 3 bereitgestellt wird, verglichen wird.

14. Verfahren, um unerwünschte Zellproliferation, die durch die MAPKs vermittelt wird, in kultivierten Zellen zu inhibieren, indem eine wirksame Menge eines Peptids nach einem der Ansprüche 1 bis 5 verabreicht wird.

15. Verwendung eines Peptids nach einem der Ansprüche 1 bis 7 zur Herstellung eines Medikaments zur Behandlung von Krebs.

16. Verwendung nach Anspruch 15, wobei der Krebs ausgewählt ist aus der Gruppe bestehend aus Karzinomen, hämatopoetischen Tumoren aus lymphoider/myeloider Linie, Lymphomen, lymphozytischer Leukämie, Tumoren mesenchymalen Ursprungs und Melanomen.

17. Verwendung eines Peptids nach einem der Ansprüche 1 bis 7 zur Herstellung eines Medikaments zur Behandlung von Autoimmun- oder inflammatorischen Erkrankungen.

18. Verwendung nach Anspruch 17, wobei die Autoimmun- oder inflammatorische Erkrankung ausgewählt ist aus der Gruppe bestehend aus Systemischem Lupus erythematodes (SLE), Typ 1-Diabetes, Gefäßentzündung, autoimmuner chronisch-aktiver Hepatitis, Colitis ulcerosa, Morbus Crohn, allergischen Erkrankungen, nephritischem Syndrom, Sarkoidose und rheumatoider Arthritis.

19. Verwendung eines Peptids nach einem der Ansprüche 1 bis 7 zur Herstellung eines Medikaments für Allotransplantat- oder Xenotransplantat-Empfänger, um die Abstoßung eines transplantierten Organs zu verhindern.

20. Peptid bestehend aus der Aminosäuresequenz SEQ ID NO:1 oder SEQ ID NO:2 zur Verwendung als ein Medikament.

21. Verfahren zum *in vitro* und *in vivo* Screening von Verbindungen, die möglicherweise an Raf binden und den MAPKs-Weg inhibieren, indem die Wirkung derartiger Verbindungen mit der Wirkung, die durch die Peptide bestehend aus der Aminosäuresequenz SEQ ID NO:1 oder SEQ ID NO:2 bereitgestellt wird, verglichen wird.

22. Kit zum *in vitro* und *in vivo* Screening von Verbindungen, die möglicherweise an Raf binden und den MAPKs-Weg inhibieren, indem die Wirkung derartiger Verbindungen mit der Wirkung, die durch die Peptide bestehend aus der Aminosäuresequenz SEQ ID NO:1 oder SEQ ID NO:2 bereitgestellt wird, verglichen wird.

23. Verwendung eines Peptids bestehend aus der Aminosäuresequenz SEQ ID NO:1 oder SEQ ID NO:2 zur Herstellung eines Medikaments zur Behandlung von Krebs.

24. Verwendung nach Anspruch 23, wobei der Krebs ausgewählt ist aus der Gruppe bestehend aus Karzinomen, hämatopoetischen Tumoren aus lymphoider/myeloider Linie, Lymphomen, lymphozytischer Leukämie, Tumoren mesenchymalen Ursprungs und Melanomen.

25. Verwendung eines Peptids bestehend aus der Aminosäuresequenz SEQ ID NO:1 oder SEQ ID NO:2 zur Herstellung eines Medikaments zur Behandlung von Autoimmun- oder inflammatorischen Erkrankungen.

26. Verwendung nach Anspruch 25, wobei die Autoimmun- oder inflammatorische Erkrankung ausgewählt ist aus der Gruppe bestehend aus Systemischem Lupus erythematodes (SLE), Typ 1-Diabetes, Gefäßentzündung, autoimmuner chronisch-aktiver Hepatitis, Colitis ulcerosa, Morbus Crohn, allergischen Erkrankungen, nephritischem Syndrom, Sarkoidose und rheumatoider Arthritis.

27. Verwendung eines Peptids bestehend aus der Aminosäuresequenz SEQ ID NO:1 oder SEQ ID NO:2 zur Herstellung eines Medikaments für Allotransplantat- oder Xenotransplantat-Empfänger, um die Abstoßung eines transplantierten Organs zu verhindern.

## Revendications

1. Peptide capable de se lier à une protéine Raf et d'inhiber la voie des MAPK, choisi parmi :
a) un peptide qui consiste en la séquence des acides aminés n° 1 à 50 de la Séquence N° 1 ou de la Séquence N° 2 ;
b) un fragment d'un peptide défini en (a), comportant au moins 10 résidus d'acides aminés, et de préférence, au moins 20 résidus d'acides aminés ;
c) et un fragment défini en (b), doté d'une séquence correspondant aux résidus d'acides aminés n° 1 à 20, n° 21 à 50, n° 10 à 30 ou n° 10 à 50 de la Séquence N° 1 ou de la Séquence N° 2.

2. Peptide capable de se lier à une protéine Raf et d'inhiber la voie des MAPK, choisi parmi :
a) un peptide qui consiste en la séquence d'acides aminés donnée en tant que Séquence N° 3 ;
b) un fragment d'un peptide défini en (a), comportant au moins 5 résidus consécutifs d'acides aminés de la Séquence N° 3 ;
c) et un fragment défini en (b), qui consiste en une séquence correspondant aux résidus d'acides aminés n° 1 à 7 ou n° 15 à 21 de la Séquence N° 3.

3. Peptide conforme à la revendication 1 ou 2, se présentant sous forme de fractions actives, de précurseurs, de sels ou de dérivés, lesquels dérivés peuvent être préparés à partir des groupes fonctionnels présents sur les chaînes latérales des résidus d'acides aminés ou à partir des groupes amino ou carboxyle terminaux.

4. Peptide mimétique des peptides conformes à l'une des revendications 1 à 3.

5. Peptide conforme à l'une des revendications 1 à 4, chimiquement modifié avec des molécules qui, parce qu'elles sont transportées naturellement à travers les membranes cellulaires, en facilitent l'entrée dans les cellules ou en améliorent l'aptitude à traverser les membranes cellulaires et à pénétrer dans le cytoplasme.

6. Séquence d'ADN codant un peptide dérivé de GILZ, conforme à la revendication 1 ou 2.

7. Vecteur d'expression comprenant un ADN conforme à la revendication 6, accompagné de n'importe quels éléments appropriés servant au démarrage ou à l'arrêt de la transcription ou de la traduction, ainsi que de n'importe quelle autre séquence supplémentaire.

8. Peptide, ADN ou vecteur conforme à l'une des revendications 1 à 7, conçu pour servir de médicament.

9. Composition pharmaceutique comprenant un composté conforme à l'une des revendications 1 à 8, en tant qu'ingrédient actif, ainsi qu'un véhicule, excipient, stabilisant ou diluant pharmacologiquement admissible.

10. Cellule hôte transformée à l'aide d'un vecteur d'expression conforme à la revendication 7.

11. Préparation purifiée d'un peptide conforme à l'une des revendications 1 à 5, contenant au moins 1 % de ce composé.

12. Procédé de criblage *in vitro* de composés susceptibles de se lier à une protéine Raf et d'inhiber la voie des MAPK, dans lequel procédé l'on compare l'effet de ces composés à l'effet de peptides conformes à l'une des revendications 1 à 3.

13. Trousse conçue pour un criblage *in vitro* ou *in vivo* de composés susceptibles de se lier à une protéine Raf et d'inhiber la voie des MAPK, criblage effectué par comparaison de l'effet de ces composés à l'effet de peptides conformes à l'une des revendications 1 à 3.

14. Procédé permettant d'inhiber, chez des cellules en culture, une prolifération cellulaire médiée par MAPK indésirable, dans lequel procédé l'on met lesdites cellules en présence d'une quantité efficace d'un peptide conforme à l'une des revendications 1 à 5.

15. Emploi d'un peptide, ADN ou vecteur, conforme à l'une des revendications 1 à 7, en vue de la fabrication d'un médicament conçu pour le traitement d'un cancer.

16. Emploi conforme à la revendication 15, le cancer étant choisi parmi les suivants : carcinomes, tumeurs hématopoïétiques de lignée lymphoïde ou myéloïde, lymphomes, leucémie lymphocytaire, tumeurs d'origine mésenchymateuse et mélanome.

17. Emploi d'un peptide, ADN ou vecteur, conforme à l'une des revendications 1 à 7, en vue de la fabrication d'un médicament conçu pour le traitement d'une maladie auto-immune ou d'une maladie inflammatoire.

18. Emploi conforme à la revendication 17, la maladie auto-immune ou la maladie inflammatoire étant choisie parmi les suivantes : lupus érythémateux aigu disséminé (LEAD), diabète de type 1, vasculite, hépatite chronique active auto-immune, colite ulcéreuse, maladie de Crohn, maladies allergiques, syndrome néphrotique, sarcoïdose et polyarthrite rhumatoïde.

19. Emploi d'un peptide, ADN ou vecteur, conforme à l'une des revendications 1 à 7, en vue de la fabrication d'un médicament conçu pour prévenir le rejet d'un organe transplanté, chez des receveurs d'une allogreffe ou d'une xénogreffe.

20. Peptide consistant en la séquence d'acides aminés donnée en tant que Séquence N° 1 ou Séquence N° 2, conçu pour servir de médicament.

21. Procédé de criblage *in vitro* de composés susceptibles de se lier à une protéine Raf et d'inhiber la voie des MAPK, dans lequel procédé l'on compare l'effet de ces composés à l'effet d'un peptide consistant en la séquence d'acides aminés donnée en tant que Séquence N° 1 ou Séquence N° 2.

22. Trousse conçue pour un criblage *in vitro* ou *in vivo* de composés susceptibles de se lier à une protéine Raf et d'inhiber la voie des MAPK, criblage effectué par comparaison de l'effet de ces composés à l'effet d'un peptide consistant en la séquence d'acides aminés donnée en tant que Séquence N° 1 ou Séquence N° 2.

23. Emploi d'un peptide consistant en la séquence d'acides aminés donnée en tant que Séquence N° 1 ou Séquence N° 2, en vue de la fabrication d'un médicament conçu pour le traitement d'un cancer.

24. Emploi conforme à la revendication 23, le cancer étant choisi parmi les suivants : carcinomes, tumeurs hématopoïétiques de lignée lymphoïde ou myéloïde, lymphomes, leucémie lymphocytaire, tumeurs d'origine mésenchymateuse et mélanome.

25. Emploi d'un peptide consistant en la séquence d'acides aminés donnée en tant que Séquence N° 1 ou Séquence N° 2, en vue de la fabrication d'un médicament conçu pour le traitement d'une maladie auto-immune ou d'une maladie inflammatoire.

26. Emploi conforme à la revendication 25, la maladie auto-immune ou la maladie inflammatoire étant choisie parmi les suivantes : lupus érythémateux aigu disséminé (LEAD), diabète de type 1, vasculite, hépatite chronique active auto-immune, colite ulcéreuse, maladie de Crohn, maladies allergiques, syndrome néphrotique, sarcoïdose et polyarthrite rhumatoïde.

27. Emploi d'un peptide consistant en la séquence d'acides aminés donnée en tant que Séquence N° 1 ou Séquence N° 2, en vue de la fabrication d'un médicament conçu pour prévenir le rejet d'un organe transplanté, chez des receveurs d'une allogreffe ou d'une xénogreffe.
